# EUROPEAN PATENT APPLICATION

(11) **EP 1 477 167 A1**
(43) Date of publication of application: **17.11.2004**
(21) Application number: 03010944.1
(22) Date of filing: 15.05.2003
(51) Int. Cl.: A61K 31/4402, C07C 237/20, C07D 213/40, C07C 311/16, A61K 31/164

(54) **[(2-hydroxy-2-(4-hydroxy-3-hydroxymethylphenyl)-ethylamino)-propyl] phenyl derivatives as beta2 agonists**

(71) Applicant: Pfizer Limited, Sandwich, Kent CT13 9NJ (GB)
(72) Inventor: Glossop, Paul, Sandwich, Kent, CT13 HR (GB); Lane, Charlotte, Tonbridge, Kent, TN9 2YR (GB); Lewthwaite, Russell, Kent, CT5 3RF (GB); Bunnage, Mark, Nr. Canterbury, Kent, CT3 1DT (GB)
(74) Representative: Hirsch, Denise Marie

(57) **Abstract**

The invention relates to compounds of formula (1) 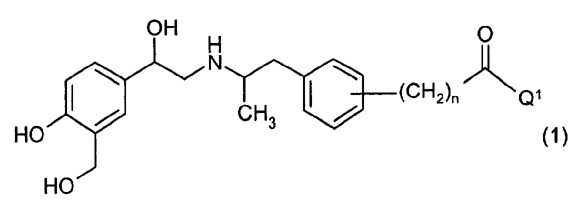 and to processes for the preparation of, intermediates used in the preparation of, compositions containing and the uses of, such derivatives. The compounds according to the present invention are useful in numerous diseases, disorders and conditions, in particular inflammatory, allergic and respiratory diseases, disorders and conditions.

## Description

This invention relates to β2 agonists of general formula: 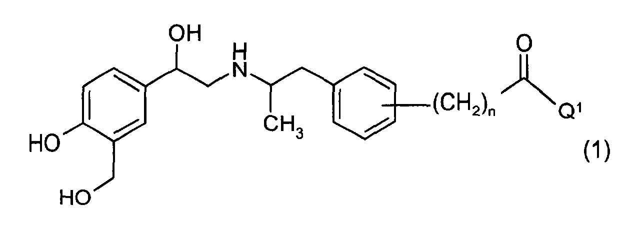 in which n and Q¹ have the meanings indicated below,
and to processes for the preparation of, intermediates used in the preparation of, compositions containing and the uses of, such derivatives.

Adrenoceptors are members of the large G-protein coupled receptor super-family. The adrenoceptor subfamily is itself divided into the α and β subfamilies with the β sub-family being composed of at least 3 receptor sub-types: β1, β2 and β3. These receptors exhibit differential expression patterns in tissues of various systems and organs of mammals. β2 adrenergic (β2) receptors are mainly expressed in smooth muscle cells (e.g. vascular, bronchial, uterine or intestinal smooth muscles), whereas β3 adrenergic receptors are mainly expressed in fat tissues (therefore β3 agonists could potentially be useful in the treatment of obesity and diabetes) and β1 adrenergic receptors are mainly expressed in cardiac tissues (therefore β1 agonists are mainly used as cardiac stimulants).

The pathophysiology and treatments of airway diseases have been extensively reviewed in the literature (for reference see Barnes, P.J. Chest, 1997, 111:2, pp 17S-26S and Bryan, S.A. et al, Expert Opinion on investigational drugs, 2000, 9:1, pp25-42) and therefore only a brief summary will be included here to provide some background information.

Glucocorticosteroids, anti-leukotrienes, theophylline, cromones, anticholinergics and β2 agonists constitute drug classes that are currently used to treat allergic and non-allergic airways diseases such as asthma and chronic obstructive airways disease (COPD). Treatment guidelines for these diseases include both short and long acting inhaled β2 agonists. Short acting, rapid onset β2 agonists are used for "rescue" bronchodilation, whereas, long-acting forms provide sustained relief and are used as maintenance therapy.

Bronchodilation is mediated via agonism of the β2 adrenoceptor expressed on airway smooth muscle cells, which results in relaxation and hence bronchodilation. Thus, as functional antagonists, β2 agonists can prevent and reverse the effects of all bronchoconstrictor substances, including leukotriene D4 (LTD4), acetylcholine, bradykinin, prostaglandins, histamine and endothelins. Because β2 receptors are so widely distributed in the airway, β2 agonists may also affect other types of cells that play a role in asthma. For example, it has been reported that β2 agonists may stabilize mast cells. The inhibition of the release of bronchoconstrictor substances may be how β2 agonists block the bronchoconstriction induced by allergens, exercise and cold air. Furthermore, β2 agonists inhibit cholinergic neurotransmission in the human airway, which can result in reduced cholinergic-reflex bronchoconstriction.

In addition to the airways, it has also been established that β2 adrenoceptors are also expressed in other organs and tissues and thus β2 agonists, such as those described in the present invention, may have application in the treatment of other diseases such as, but not limited to those of the nervous system, premature labor, congestive heart failure, depression, inflammatory and allergic skin diseases, psoriasis, proliferative skin diseases, glaucoma and in conditions where there is an advantage in lowering gastric acidity, particularly in gastric and peptic ulceration.

However, numerous β2 agonists are limited in their use due to their low selectivity or adverse side-effects driven by high systemic exposure and mainly mediated through action at β2 adrenoreceptors expressed outside the airways (muscle tremor, tachycardia, palpitations, restlessness). Therefore there is a need for improved agents in this class.

Accordingly, there is still a need for novel β2 agonists that would have an appropriate pharmacological profile, for example in terms of potency, selectivity and/or pharmacodynamic properties. In this context, the present invention relates to novel β2 agonists.
EP 0654534 B1 and EP0939134 B1 disclose a process for the preparation of compounds of formula (XI): 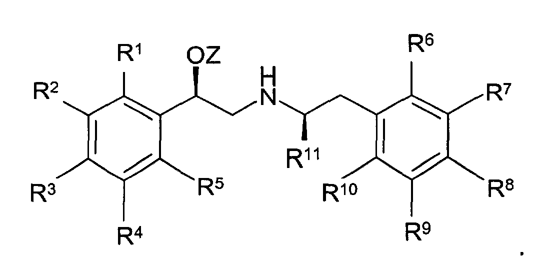 These compounds are disclosed as anti-obesity and anti-diabetic agents having specific β3 activity.
US5,561,142 discloses selective β3 agonists of formula 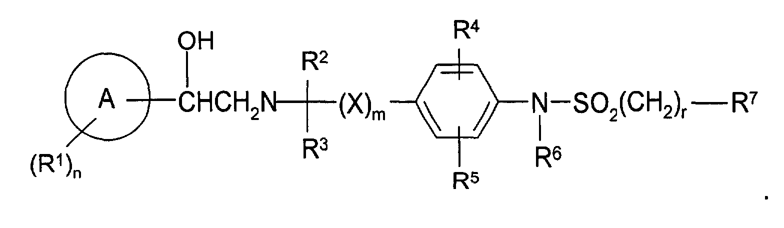 EP0236624 discloses compounds of formula 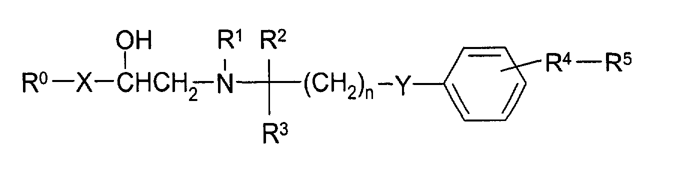 having anti-obesity and/or anti-hyperglycaemic activity coupled with good selectivity from cardiac side-effects.

The invention relates to compounds of general formula (1): 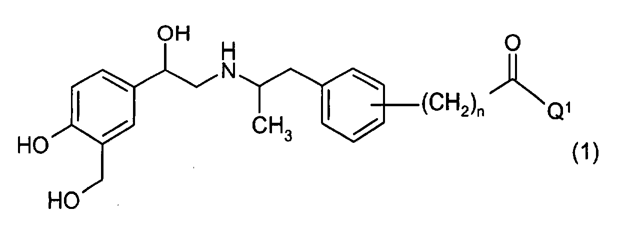 wherein the (CH₂)ₙ-C(=O)Q¹ group is in position meta or para, n is 1 or 2 and Q¹ is a group selected from: 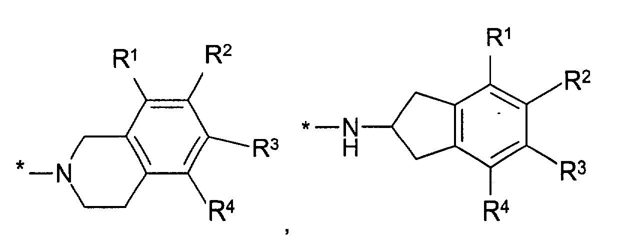 and a group ^{*}-NH-Q²-A, wherein Q² is C₁-C₄ alkylene and A is pyridyl or a group of formula 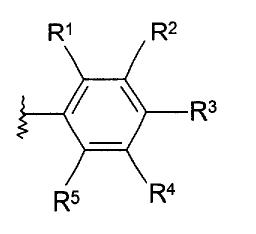 wherein R¹, R², R³, R⁴ and R⁵ are the same or different and are selected from H, C₁-C₄ alkyl, OR⁶, SR⁶, halo, CF₃, OCF₃, COOR⁶, SO₂NR⁶R⁷, CONR⁶R⁷, NR⁶R⁷, NHCOR⁶, wherein at least 2 of R₁ to R₅ are equal to H;
wherein R⁶ and R⁷ are the same or different and are selected from H or C₁-C₄ alkyl and the * represent the attachment point to the carbonyl group;
or, if appropriate, their pharmaceutically acceptable salts and/or isomers, tautomers, solvates or isotopic variations thereof.
It has now been found that the compounds of formula (1) are agonists of the β2 receptors, that are particularly useful for the treatment of β2-mediated diseases and/or conditions, and show good potency, in particular when administered via the inhalation route.

In the present invention, the term "potent" means that the compounds of formula (1) show an agonist potency for the β2 receptor, which is less than 10 nM as measured by the cell-based assay described herein.
Preferably, the compounds of the invention are selective agonists of the β2 receptor receptors. Preferably, the compounds of the invention show an agonist potency for the β2 receptor, which is at least about 100-fold higher as for the β3 receptor and at least about 500-fold higher as for the β1 receptor.

In the here above general formula (1), C₁-C₄ alkyl and C₁-C₄ alkylene denote a straight-chain or branched group containing 1, 2, 3 or 4 carbon atoms. This also applies if they carry substituents or occur as substituents of other radicals, for example in O-(C₁-C₄)alkyl radicals, S-(C₁-C₄)alkyl radicals etc .... Examples of suitable (C₁-C₄)alkyl radicals are methyl, ethyl, *n*-propyl, *iso*-propyl, *n*-butyl, *iso*-butyl, *sec*-butyl, *tert*-butyl.... Examples of suitable (C₁-C₄)alkoxy radicals are methoxy, ethoxy, *n*-propyloxy, *iso*-propyloxy, *n*-butyloxy, *iso*-butyloxy, *sec*-butyloxy and *tert*-butyloxy....

Finally, halo denotes a halogen atom selected from the group consisting of fluoro, chloro, bromo and iodo in particular fluoro or chloro.
In the following, the free bond on the phenyl group such as in the structure below, 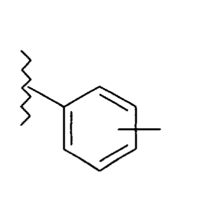 means that the phenyl can be substituted in the meta or para position.

The compounds of the formula (1) 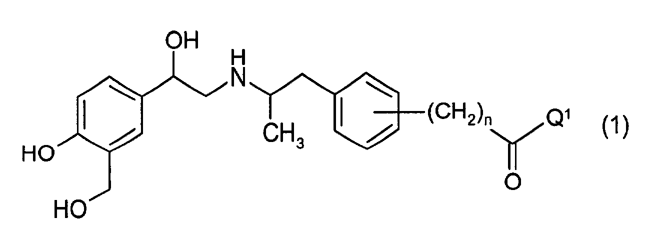 can be prepared using conventional procedures such as by the following illustrative methods in which Q¹, Q², A and n are as previously defined for the compounds of the formula (1) unless otherwise stated.

The amide derivatives of the formula (1) may be prepared by coupling an acid of formula (2): 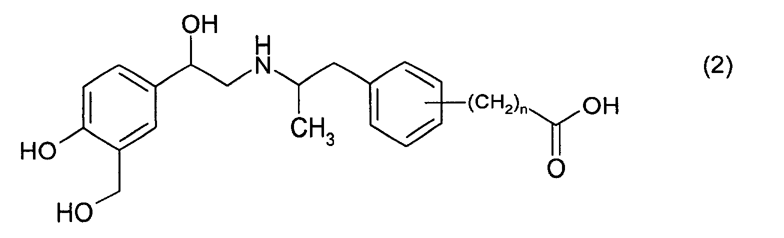 with an amine of formula NH₂-Q²-A (3), 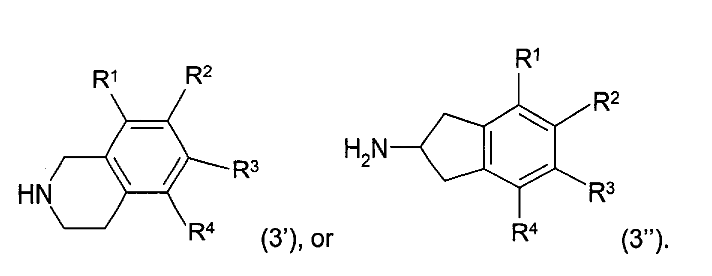 The coupling is generally carried out in an excess of said amine as an acid receptor, with a conventional coupling agent (e.g. 1-(3-dimethylaminopropyl)-3-ethylcarbodiimide hydrochloride or *N, N'*-dicyclohexylcarbodiimide), optionally in the presence of a catalyst (e.g. 1-hydroxybenzotriazole hydrate or 1-hydroxy-7-azabenzotriazole), and optionally in the presence of a tertiary amine base (e.g. N-methylmorpholine, triethylamine or diisopropylethylamine). The reaction may be undertaken in a suitable solvent such as pyridine, dimethylformamide, tetrahydrofuran, dimethylsulfoxide, dichloromethane or ethyl acetate, and at temperature comprised between 10°C and 40°C (room temperature) for a period of 1-24 hours.

Said amine (3), (3') or (3") is either commercially available or may be prepared by conventional methods well known to the one skilled in the art (e.g. reduction, oxidation, alkylation, protection, deprotection etc...) from commercially available material.

The acid of formula (2) may be prepared from the corresponding ester of formula (4) : 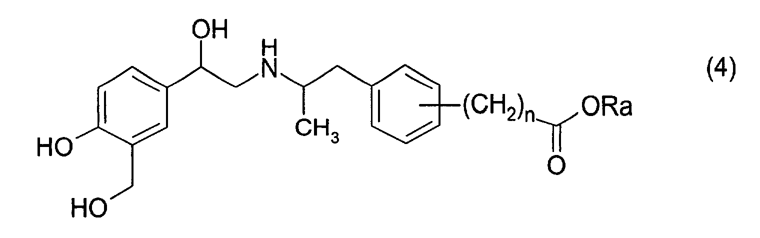 wherein Ra is a suitable acid protecting group, preferably a (C₁-C₄)alkyl group, which includes, but is not limited to, methyl and ethyl, according to any method well-known to the one skilled in the art to prepare an acid from an ester, without modifying the rest of the molecule. For example, the ester may be hydrolysed by treatment with aqueous acid or base (e.g. hydrogen chloride, potassium hydroxide, sodium hydroxide or lithium hydroxide), optionally in the presence of a solvent or mixture of solvents (e.g. water, 1,4-dioxan, tetrahydrofuran/water), at a temperature comprised between 20°C and 100°C, for a period of 1 to 40 hours.

The ester of formula (4) may be prepared by reaction of an amine of formula (5) : 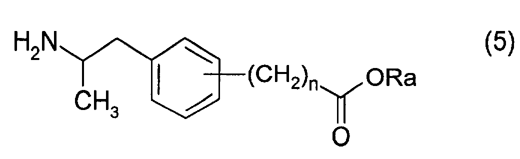 wherein Ra and n are as previously defined, with a bromide of formula (6) :

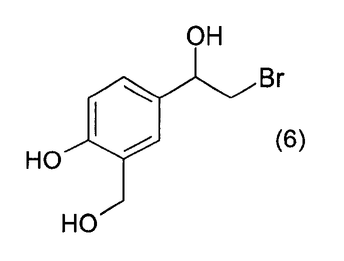

In a typical procedure, the amine of formula (5) is reacted with a bromide of formula (6) optionally in the presence of a solvent or mixture of solvents (e.g. dimethyl sulphoxide, toluene, *N, N*-dimethylformamide, acetonitrile), optionally in the presence of a suitable base (e.g. triethylamine, diisopropylethylamine, potassium carbonate) at a temperature comprised between 80°C and 120°C, for 12 to 48 hours.

The bromide of formula (6) may be prepared from the ester of formula (7) : 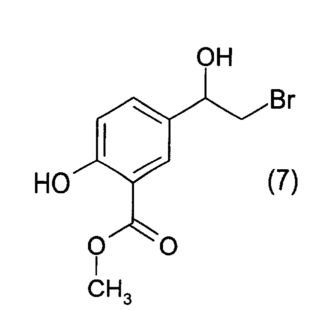 according to any method well-known to the one skilled in the art to prepare an alcohol from an ester, without modifying the rest of the molecule.

In a typical procedure, the ester of formula (7) is reduced with borane dimethylsulfide complex in tetrahydrofuran at a reflux for a period of 2 hours.

The alcohol of formula (7) may be prepared as either the (*R*) or (*S*) enantiomer according to methods well described in the literature (Tetrahedron Letters 1994, 35(50), 9375).

The amine of formula (5) may be prepared as either the (*R*) or (*S*) enantiomer from the corresponding protected amine of formula (8) : 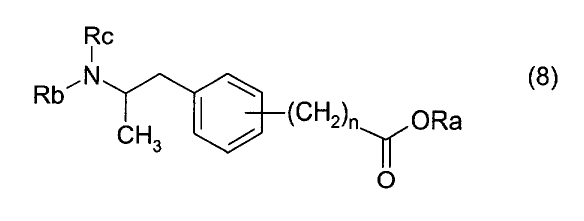 wherein Ra and n are as previously defined and Rb and Rc represent any suitable substituents so that HNRbRc is a chiral amine (for example, Rb may be hydrogen and Rc may be α-methylbenzyl), provided that the bonds between N and Rb and N and Rc can be easily cleaved to give the free amine of formula (5) using standard methodology for cleaving nitrogen protecting groups, such as those found in the text book T.W. GREENE, Protective Groups in Organic Synthesis, A. Wiley-Interscience Publication, 1981.

The amine of formula (8) may be prepared as a single diastereomer by reaction of an amine of formula HNRbRc with a ketone of formula (9): 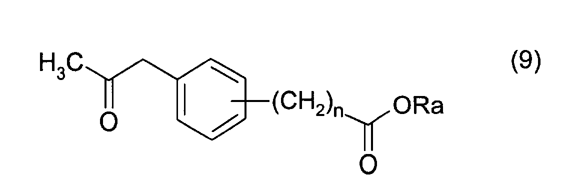 wherein Ra, Rb, Rc and n are as previously defined.

In a typical procedure, the reaction of the ketone of formula (9) with the amine of formula HNRbRc leads to a chiral intermediate which is in turn reduced by a suitable reducing agent (e.g. sodium cyanoborohydride of formula NaCNBH₃ or sodium triacetoxyborohydride of formula Na(OAc)₃BH) optionally in the presence of a drying agent (e.g. molecular sieves, magnesium sulfate) and optionally in the presence of an acid catalyst (e.g. acetic acid) to give the amine of formula (8) as a mixture of diastereomers. The reaction is generally done in a solvent such as tetrahydrofuran or dichloromethane at a temperature comprised between 20°C and 80°C for 3 to 72 hours. The resulting product is then converted to the hydrochloride salt and selectively crystallised from a suitable solvent or mixture of solvents (e.g. isopropanol, ethanol, methanol, diisopropyl ether or diisopropyl ether/methanol) to give (8) as a single diastereomer.

The ketone of formula (9) where n=1 may be prepared by palladium mediated coupling of an aryl halide of formula (10): 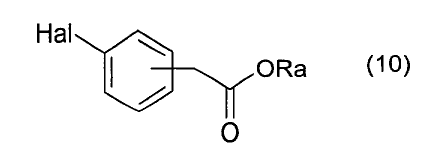 wherein Ra is as previously defined and Hal represents an halogen atom, which includes, but is not limited to bromo and iodo, with an enolate or enolate equivalent.

In a typical procedure, the aryl halide of formula (10) is reacted with a tin enolate generated in-situ by treatment of isoprenyl acetate with tri-n-butyltin methoxide of formula Bu₃SnOMe in the presence of a suitable palladium catalyst (palladium acetate/ tri-*ortho*-tolylphosphine of formula Pd(OAc)₂/P(o-Tol)₃) in a non-polar solvent (e.g. toluene, benzene, hexane). Preferably, the reaction is carried out at a temperature comprised between 80°C and 110°C for 6 to 16 hours.

The aryl halide of formula (10) may be obtained by esterification of the corresponding acid of formula (11): 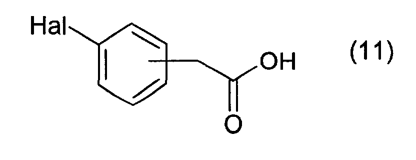 wherein Hal is as previously defined,
according to any method well-known to the one skilled in the art to prepare an ester from an acid, without modifying the rest of the molecule.

In a typical procedure, the acid of formula (11) is reacted with an alcoholic solvent of formula RaOH, wherein Ra is as previously defined, in the presence of an acid such as hydrogen chloride at a temperature between 10°C and 40°C (room temperature) for 8 to 16 hours.

The acid of formula (11) is a commercial product.

The ketone of formula (9) where n=2 may be prepared by reduction of an alkene of formula (12) :

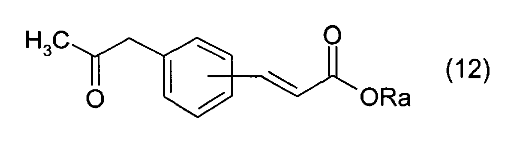

In a typical procedure, a solution of the olefin of formula (12) in a suitable solvent (e.g. methanol, ethanol, ethyl acetate) is treated with a palladium catalyst (e.g. 10% palladium on charcoal) and stirred under an atmosphere of hydrogen, optionally at elevated pressure (e.g. 60 psi), at temperature between room temperature and 60°C for 8-24 hours.

The alkene of formula (12) may be prepared by a palladium mediated coupling of an activated olefin with an aryl halide of formula (13):

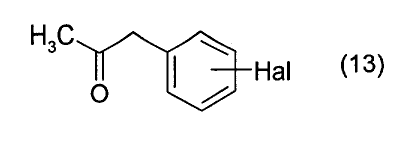

In a typical procedure, the aryl halide (13) is coupled with a vinyl ester (e.g. methyl acrylate) in the presence of a suitable palladium catalyst (e.g. tetrakis(triphenylphosphine)palladium(0) of formula Pd(PPh₃)₄, palladium acetate/tri-*ortho*-tolylphosphine of formula Pd(OAc)₂/P(o-tol)₃ or (diphenylphosphino)ferrocenyl palladium chloride of formula dppfPdCl₂) in a sutiable solvent (e.g. acetonitrile, *N, N*-dimethylformamide, toluene), optionally in the presence of a base such as triethylamine at a temperature between 40°C and 110°C for 8 to 24 hours.

The ketone of formula (13) is a commercial product.
For some of the steps of the here above described process of preparation of the compounds of formula (1), it may be necessary to protect potential reactive functions that are not wished to react, and to cleave said protecting groups in consequence. In such a case, any compatible protecting radical can be used. In particular methods of protection and deprotection such as those described by T.W. GREENE (*Protective Groups in Organic Synthesis,* A. Wiley-Interscience Publication, 1981) or by P. J. Kocienski (*Protecting groups,* Georg Thieme Verlag, 1994), can be used.

All of the above reactions and the preparations of novel starting materials used in the preceding methods are conventional and appropriate reagents and reaction conditions for their performance or preparation as well as procedures for isolating the desired products will be well-known to those skilled in the art with reference to literature precedents and the examples and preparations hereto.

Also, the compounds of formula (1) as well as intermediate for the preparation thereof can be purified according to various well-known methods, such as for example crystallization or chromatography.
Compounds of formula (1) wherein n is 1 or 2, Q¹ is a group *-NH-Q²-A, wherein Q² is C₁-C₄ alkylene and A is group of formula 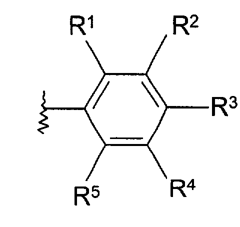 wherein R¹, R², R³, R⁴ and R⁵ are as defined above, are particularly preferred.

Preferably n is 1.
Preferably, Q² is selected from -CH₂-, -(CH₂)₂-, -(CH₂)₃- and -CH(CH₃)-.
More preferably, Q² is -CH₂-.
Preferably, R¹, R², R³, R⁴ and R⁵ are the same or different and are selected from H, C₁-C₄ alkyl, OR⁶, CI, F, CF₃, OCF₃, COOR⁶, SO₂NR⁶R⁷, provided at least 2 of R¹ to R⁵ are equal to H,
wherein R⁶ and R⁷ are the same or different and are selected from H or C₁-C₄ alkyl.

Preferably, R¹, R², R³, R⁴ and R⁵ are the same or different and are selected from H, CH₃, OH, OCH₃, OCH₂CH₃, CI, F, CF₃, OCF₃, COOH, SO₂NH₂, provided at least 2 of R¹ to R⁵ are equal to H.

Preferably, R¹, R², R³, R⁴ and R⁵ are the same or different and are selected from H, CH₃, OH, OCH₃, OCH₂CH₃, CI, F, CF₃, OCF₃, COOH, SO₂NH₂, provided at least 3 of R¹ to R⁵ are equal to H.

Preferably, R¹, R², R³, R⁴ and R⁵ are selected from CI, provided at least 3 of R¹ to R⁵ are equal to H.

Other preferred compounds are those wherein n is 1 or 2 and Q¹ is 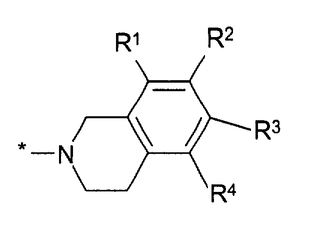 wherein R¹, R², R³ and R⁴ are the same or different and are selected from H, C₁-C₄ alkyl, OR⁶, SR⁶, halo, CF₃, OCF₃, COOR⁶, SO₂NR⁶R⁷, CONR⁶R⁷, NR⁶R⁷, NHCOR⁶, provided at least 2 of R¹ to R⁴ are equal to H;
wherein R⁶ and R⁷ are the same or different and are selected from H or C₁-C₄ alkyl.

Preferably, R¹, R², R³, and R⁴ are the same or different and are selected from H and OR⁶, provided at least 2 of R¹ to R⁴ are equal to H;

Other preferred compounds are those wherein n is 1 or 2 and Q¹ is 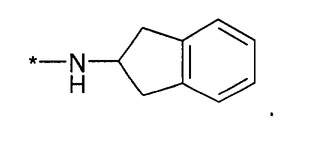 Other preferred compounds are those wherein n is 1 or 2 and Q¹ is a group *-NH-Q²-A, wherein Q² is C₁-C₄ alkylene and A is pyridin-2-yl.

Particularly preferred are the compounds of the formula (1) as described in the Examples section hereafter, i.e. :
*N*-(2,6-Dimethoxybenzyl)-2-(3-{(2*R*)-2-[(2*R*)-2-hydroxy-2-(4-hydroxy-3-hydroxymethyl-phenyl)-ethylamino]-propyl}-phenyl)-acetamide,
*N*-(2-Ethoxybenzy))-2-(3-{(2*R*)-2-[(2*R*)-2-hydroxy-2-(4-hydroxy-3-hydroxymethy)-phenyl)-ethylamino]-propyl}-phenyl)-acetamide,
*N*-(2-Hydroxybenzyl)-2-(3-{(2*R*)-2-[(2*R*)-2-hydroxy-2-(4-hydroxy-3-hydroxymethyl-phenyl)-ethylamino]-propyl}-phenyl)-acetamide,
2-(3-{(2*R*)-2-[(2*R*)-2-Hydroxy-2-(4-hydroxy-3-hydroxymethyl-phenyl)-ethylamino]-propyl}-phenyl)-*N*-indan-2-yl-acetamide,
*N*-(3,4-Dichlorobenzyl)-2-(3-{(2*R*)-2-[(2*R*)-2-hydroxy-2-(4-hydroxy-3-hydroxymethyl-phenyl)-ethylamino]-propyl}-phenyl)-acetamide,
*N*-[4-(Aminosulfonyl)benzyl]-2-(3-{(2*R*)-2-[(2*R*)-2-hydroxy-2-(4-hydroxy-3-hydroxymethyl-phenyl)-ethylamino]-propyl}-phenyl)-acetamide,
2-(3-{(2*R*)-2-[(2*R*)-2-Hydroxy-2-(4-hydroxy-3-hydroxymethyl-phenyl)-ethylamino]-propyl}-phenyl)-*N*-(pyridin-2-ylmethyl)-acetamide,
4-{(2-(3-{(2*R*)-2-[(2*R*)-2-Hydroxy-2-(4-hydroxy-3-hydroxymethyl-phenyl)-ethylamino]-propyl}-phenyl)-acetylamino)-methyl}-benzamide,
*N*-(3,4-Dimethoxybenzyl)-2-(3-{(2*R*)-2-[(2*R*)-2-hydroxy-2-(4-hydroxy-3-hydroxymethyl-phenyl)-ethylamino]-propyl}-phenyl)-acetamide,
2-(3-{(2*R*)-2-[(2*R*)-2-Hydroxy-2-(4-hydroxy-3-hydroxymethyl-phenyl)-ethylamino]-propyl}-phenyl)-*N*-(4-trifluoromethoxybenzyl)-acetamide,
*N*-(2-Chloro,6-fluorobenzyl)-2-(3-{(2*R*)-2-[(2*R*)-2-hydroxy-2-(4-hydroxy-3-hydroxymethyl-phenyl)-ethylamino]-propyl}-phenyl)-acetamide,
*N*-(3,4-Dimethylbenzyl)-2-(3-{(2*R*)-2-[(2*R*)-2-hydroxy-2-(4-hydroxy-3-hydroxymethyl-phenyl)-ethylamino]-propyl}-phenyl)-acetamide,
*N*-[2-Fluoro-5-(trifluoromethyl)benzyl]-2-(3-{(2*R*)-2-[(2*R*)-2-hydroxy-2-(4-hydroxy-3-hydroxymethyl-phenyl)-ethylamino]-propyl}-phenyl)-acetamide,
*N*-(2,6-Dichlorobenzyl)-2-(3-{(2*R*)-2-[(2*R*)-2-hydroxy-2-(4-hydroxy-3-hydroxymethyl-phenyl)-ethylamino]-propyl}-phenyl)-acetamide,
2-(3-{(2*R*)-2-[(2*R*)-2-Hydroxy-2-(4-hydroxy-3-hydroxymethyl-phenyl)-ethylamino]-propyl}-phenyl)-*N*-(2-phenylethyl)acetamide,
*N*-Benzyl-2-(3-{(2*R*)-2-[(2*R*)-2-hydroxy-2-(4-hydroxy-3-hydroxymethyl-phenyl)-ethylamino]-propyl}-phenyl)-acetamide,
*N*-(3,5-Dichlorobenzyl)-2-(3-{(2*R*)-2-[(2*R*)-2-hydroxy-2-(4-hydroxy-3-hydroxymethyl-phenyl)-ethylamino]-propyl}-phenyl)-acetamide,
*N*-(4-Chlorobenzyl)-2-(3-{(2*R*)-2-[(2*R*)-2-hydroxy-2-(4-hydroxy-3-hydroxymethyl-phenyl)-ethylamino]-propyl}-phenyl)-acetamide,
4-{([2-(3-{(2*R*)-2-[(2*R*)-2-Hydroxy-2-(4-hydroxy-3-hydroxymethyl-phenyl)-ethylamino]-propyl}-phenyl)-acetyl]-amino)-methyl}-benzoic acid,
*N*-(2,6-Dichlorobenzyl)-3-(3-{(2*R*)-2-[(2*R*)-2-hydroxy-2-(4-hydroxy-3-hydroxymethyl-phenyl)-ethylamino]-propyl}-phenyl)-propanamide,
1-(3,4-Dihydro-1H-isoquinolin-2-yl)-3-(3-{(2*R*)-2-[(2*R*)-2-hydroxy-2-(4-hydroxy-3-hydroxymethyl-phenyl)-ethylamino]-propyl}-phenyl)-propan-1-one,
1-(3,4-Dihydro-1H-isoquinolin-2-yl)-2-(3-{(2*R*)-2-[(2*R*)-2-hydroxy-2-(4-hydroxy-3-hydroxymethyl-phenyl)-ethylamino]-propyl}-phenyl)-ethanone,
1-(7-Ethoxy-6-methoxy-3,4-dihydro-1H-isoquinolin-2-yl)-2-(3-{(2*R*)-2-[(2*R*)-2-hydroxy-2-(4-hydroxy-3-hydroxymethyl-phenyl)-ethylamino]-propyl}-phenyl)-ethanone,
3-(3-{(2*R*)-2-[(2*R*)-2-Hydroxy-2-(4-hydroxy-3-hydroxymethyl-phenyl)-ethylamino]-propyl}-phenyl)-N-indan-2-yl-propanamide,
N-(3,4-Dimethylbenzyl)-3-(3-{(2*R*)-2-[(2*R*)-2-hydroxy-2-(4-hydroxy-3-hydroxymethyl-phenyl)-ethylamino]-propyl}-phenyl)-propanamide,
N-(2,6-Dimethoxybenzyl)-3-(3-{(2*R*)-2-[(2*R*)-2-hydroxy-2-(4-hydroxy-3-hydroxymethyl-phenyl)-ethylamino]-propyl}-phenyl)-propanamide,
N-[(1*R*)-1-Phenylethyl]-2-(3-{(2*R*)-2-[(2*R*)-2-hydroxy-2-(4-hydroxy-3-hydroxymethyl-phenyl)-ethylamino]-propyl}-phenyl)-acetamide,
N-[2-(3,4-Dimethoxyphenyl)ethyl]-2-(3-{(2*R*)-2-[(2*R*)-2-hydroxy-2-(4-hydroxy-3-hydroxymethyl-phenyl)-ethylamino]-propyl}-phenyl)-acetamide,
N-[2-(4-Chlorophenyl)ethyl]-2-(3-{(2*R*)-2-[(2*R*)-2-hydroxy-2-(4-hydroxy-3-hydroxymethyl-phenyl)-ethylamino]-propyl}-phenyl)-acetamide,
N-[2-(4-Ethylphenyl)ethyl]-2-(3-{(2*R*)-2-[(2*R*)-2-hydroxy-2-(4-hydroxy-3-hydroxymethyl-phenyl)-ethylamino]-propyl}-phenyl)-acetamide,
N-(1,1-Dimethyl-2-phenylethyl)-2-(3-{(2*R*)-2-[(2*R*)-2-hydroxy-2-(4-hydroxy-3-hydroxymethyl-phenyl)-ethylamino]-propyl}-phenyl)-acetamide,
2-(3-{(2*R*)-2-[(2*R*)-2-Hydroxy-2-(4-hydroxy-3-hydroxymethyl-phenyl)-ethylamino]-propyl}-phenyl)-N-[3-(4-fluorophenyl)propyl]-acetamide,
N-(3-Chlorobenzyl)-2-(4-{(2*R*)-2-[(2*R*)-2-hydroxy-2-(4-hydroxy-3-hydroxymethylphenyl)-ethylamino]-propyl}-phenyl)-acetamide,
2-(4-{(2*R*)-2-[(2*R*)-2-Hydroxy-2-(4-hydroxy-3-hydroxymethyl-phenyl)-ethylamino]-propyl}-phenyl)-N-indan-2-yl-acetamide,
N-(2-Hydroxybenzyl)-2-(4-{(2*R*)-2-[(2*R*)-2-hydroxy-2-(4-hydroxy-3-hydroxymethyl-phenyl)-ethylamino]-propyl}-phenyl)-acetamide,
2-(4-{(2*R*)-2-[(2*R*)-2-Hydroxy-2-(4-hydroxy-3-hydroxymethyl-phenyl)-ethylamino]-propyl}-phenyl)-N-(2-methoxybenzyl)-acetamide,
N-Benzyl-2-(4-{(2*R*)-2-[(2*R*)-2-hydroxy-2-(4-hydroxy-3-hydroxymethyl-phenyl)-ethylamino]-propyl}-phenyl)-acetamide,
N-(4-Chlorobenzyl)-2-(4-{(2*R*)-2-[(2*R*)-2-hydroxy-2-(4-hydroxy-3-hydroxymethyl-phenyl)-ethylamino]-propyl}-phenyl)-acetamide,
2-(4-{(2*R*)-2-[(2*R*)-2-Hydroxy-2-(4-hydroxy-3-hydroxymethyl-phenyl)-ethylamino]-propyl}-phenyl)-N-(3-methoxybenzyl)-acetamide,
2-(4-{(2*R*)-2-[(2*R*)-2-Hydroxy-2-(4-hydroxy-3-hydroxymethyl-phenyl)-ethylamino]-propyl}-phenyl)-N-(pyridin-2-ylmethyl)-acetamide,
2-(4-{(2*R*)-2-[(2*R*)-2-Hydroxy-2-(4-hydroxy-3-hydroxymethyl-phenyl)-ethylamino]-propyl}-phenyl)-N-(4-methoxybenzyl)-acetamide,
2-(4-{(2*R*)-2-[(2*R*)-2-Hydroxy-2-(4-hydroxy-3-hydroxymethyl-phenyl)-ethylamino]-propyl}-phenyl)-N-(3-phenylpropyl)-acetamide,
N-(2,6-Dimethoxybenzyl)-2-(4-{(2*R*)-2-[(2*R*)-2-hydroxy-2-(4-hydroxy-3-hydroxymethyl-phenyl)-ethylamino]-propyl}-phenyl)-acetamide, and,
1-(3,4-Dihydro-1H-isoquinolin-2-yl)-2-(4-{(2*R*)-2-[(2*R*)-2-hydroxy-2-(4-hydroxy-3-hydroxymethyl-phenyl)-ethylamino]-propyl}-phenyl)-ethanone.

According to one aspect of the present invention, the compounds of formula (1) wherein the (CH₂)ₙ-C(=O)Q¹ group is in position meta are generally preferred.

The compounds of formula (1) may also be optionally transformed into pharmaceutically acceptable salts. In particular, these pharmaceutically acceptable salts of the compounds of the formula (1) include the acid addition and the base salts (including disalts) thereof.

Suitable acid addition salts are formed from acids which form non-toxic salts. Examples include the acetate, aspartate, benzoate, besylate, bicarbonate/carbonate, bisulphate, camsylate, citrate, edisylate, esylate, fumarate, gluceptate, gluconate, glucuronate, hibenzate, hydrochloride/chloride, hydrobromide/bromide, hydroiodide/iodide, hydrogen phosphate, isethionate, D- and L-lactate, malate, maleate, malonate, mesylate, methylsulphate, 2-napsylate, nicotinate, nitrate, orotate, palmoate, phosphate, saccharate, stearate, succinate sulphate, D- and L-tartrate, 1-hydroxy-2-naphthoate and tosylate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

For a review on suitable salts, see Stahl and Wermuth, Handbook of Pharmaceutical Salts: Properties, Selection, and Use, Wiley-VCH, Weinheim, Germany (2002).

A pharmaceutically acceptable salt of a compound of formula (1) may be readily prepared by mixing together solutions of the compound of formula (1) and the desired acid or base, as appropriate. The salt may precipitate from solution and be collected by filtration or may be recovered by evaporation of the solvent.

Pharmaceutically acceptable solvates in accordance with the invention include hydrates and solvates wherein the solvent of crystallization may be isotopically substituted, *e.g.* D₂O, d₆-acetone, d₆-DMSO.

Also within the scope of the invention are clathrates, drug-host inclusion complexes wherein, in contrast to the aforementioned solvates, the drug and host are present in non-stoichiometric amounts. For a review of such complexes, see J Pharm Sci, 64 (8), 1269-1288 by Haleblian (August 1975).

Hereinafter all references to compounds of formula (1) include references to salts thereof and to solvates and clathrates of compounds of formula (1) and salts thereof.

The invention includes all polymorphs of the compounds of formula (1) as hereinbefore defined.

Also within the scope of the invention are so-called "prodrugs" of the compounds of formula (1). Thus certain derivatives of compounds of formula (1) which have little or no pharmacological activity themselves can, when metabolised upon administration into or onto the body, give rise to compounds of formula (1) having the desired activity. Such derivatives are referred to as "prodrugs".

Prodrugs in accordance with the invention can, for example, be produced by replacing appropriate functionalities present in the compounds of formula (1) with certain moieties known to those skilled in the art as "pro-moieties" as described, for example, in "Design of Prodrugs" by H Bundgaard (Elsevier, 1985).

Finally, certain compounds of formula (1) may themselves act as prodrugs of other compounds of formula (1).
The present invention also includes all pharmaceutically acceptable isotopic variations of a compound of formula (1). An isotopic variation is defined as one in which at least one atom is replaced by an atom having the same atomic number, but an atomic mass different from the atomic mass usually found in nature.

Examples of isotopes suitable for inclusion in the compounds of the invention include isotopes of hydrogen, such as ²H and ³H, carbon, such as ¹³C and ¹⁴C, nitrogen, such as ¹⁵N, oxygen, such as ¹⁷O and ¹⁸O, phosphorus, such as ³²P, sulphur, such as ³⁵S, fluorine, such as ¹⁸F, and chlorine, such as ³⁶Cl.

Substitution of the compounds of the invention with isotopes such as deuterium, *i.e.* ²H, may afford certain therapeutic advantages resulting from greater metabolic stability, for example, increased *in vivo* half-life or reduced dosage requirements, and hence may be preferred in some circumstances.

Certain isotopic variations of the compounds of formula (1), for example, those incorporating a radioactive isotope, are useful in drug and/or substrate tissue distribution studies. The radioactive isotopes tritium, *i.e.* ³H, and carbon-14, *i.e.* ¹⁴C, are particularly useful for this purpose in view of their ease of incorporation and ready means of detection.

Isotopic variations of the compounds of formula (1) can generally be prepared by conventional techniques known to those skilled in the art or by processes analogous to those described in the accompanying Examples and Preparations using appropriate isotopic variations of suitable reagents.

The compounds of the formula (1) can also be present in stereoisomeric forms. If the compounds of the formula (1) contain one or more centres of asymmetry, these can independently of one another have the (S) configuration or the (R) configuration. The invention includes all possible stereoisomers of the compounds of the formula (1), for example enantiomers and diastereomers, and mixtures of two or more stereoisomeric forms, for example mixtures of enantiomers and/or diastereomers, in all ratios. The invention thus relates to enantiomers in enantiomerically pure form, both as levorotatory and dextrorotatory antipodes, in the form of racemates and in the form of mixtures of the two enantiomers in all ratios. The invention likewise relates to diastereomers in diastereomerically pure form and in the form of mixtures in all ratios. In the presence of cis/trans isomerism, the invention relates to both the cis form and the trans form and mixtures of these forms in all ratios. Individual stereoisomers can be prepared, if desired, by use of stereochemically homogeneous starting substances in the synthesis, by stereoselective synthesis or by separation of a mixture according to customary methods, for example by chromatography, crystallization or by chromatography on chiral phases. If appropriate, derivatization can be carried out before separation of stereoisomers. A stereoisomer mixture can be separated at the stage of the compounds of the formula (1) or at the stage of a starting substance or of an intermediate in the course of the synthesis.

According to one aspect of the present invention, the (*R,R*)-stereoisomer of the formula below is generally preferred:

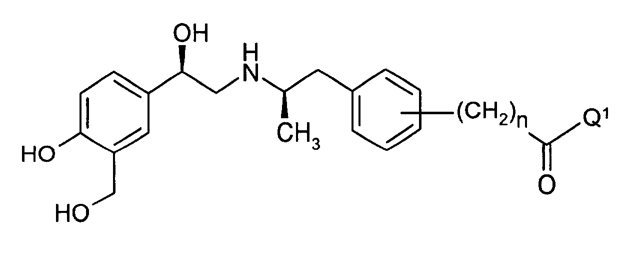

The compounds of the formula (1) according to the invention can moreover contain mobile hydrogen atoms, i.e. be present in various tautomeric forms. The present invention also relates to all tautomers of the compounds of the formula (1).

According to a further aspect, the present invention concerns mixtures of compounds of the formula (1), as well as mixtures with or of their pharmaceutically acceptable salts, solvates, isomeric forms and/or isotope forms.

According to the present invention, all the here above mentioned forms of the compounds of formula (1) except the pharmaceutically acceptable salts (i.e. said solvates, isomeric forms, tautomers and isotope forms), are defined as "derived forms" of the compounds of formula (1) in what follows (including the claims).

The compounds of formula (1), their pharmaceutically acceptable salts and/or derived forms, are valuable pharmaceutically active compounds, which are suitable for the therapy and prophylaxis of numerous disorders in which the β2 receptor is involved or in which agonism of this receptor may induce benefit, in particular the allergic and non-allergic airways diseases but also in the treatment of other diseases such as, but not limited to those of the nervous system, premature labor, congestive heart failure, depression, inflammatory and allergic skin diseases, psoriasis, proliferative skin diseases, glaucoma and in conditions where there is an advantage in lowering gastric acidity, particularly in gastric and peptic ulceration.

The compounds of formula (1) and their pharmaceutically acceptable salts and derived forms as mentioned above can be administered according to the invention to animals, preferably to mammals, and in particular to humans, as pharmaceuticals for therapy and/or prophylaxis. They can be administered per se, in mixtures with one another or in the form of pharmaceutical preparations which as active constituent contain an efficacious dose of at least one compounds of formula (1), its pharmaceutically acceptable salts and/or derived forms, in addition to customary pharmaceutically innocuous excipients and/or additives.

The compounds of formula (1), their pharmaceutically acceptable salts and/or derived forms may be freeze-dried, spray-dried, or evaporatively dried to provide a solid plug, powder, or film of crystalline or amorphous material. Microwave or radio frequency drying may be used for this purpose.

The compounds of formula (1), their pharmaceutically acceptable salts and/or derived forms may be administered alone or in combination with other drugs and will generally be administered as a formulation in association with one or more pharmaceutically acceptable excipients. The term "excipient" is used herein to describe any ingredient other than the compound of the invention. The choice of excipient will to a large extent depend on the particular mode of administration.

### ORAL ADMINISTRATION

The compounds of formula (1), their pharmaceutically acceptable salts and/or derived forms may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi- and nano-particulates, gels, films (including muco-adhesive), ovules, sprays and liquid formulations.

Liquid formulations include suspensions, solutions, syrups and elixirs. Such formulations may be employed as fillers in soft or hard capsules and typically comprise a carrier, for example, water, ethanol, propylene glycol, methylcellulose, or a suitable oil, and one or more emulsifying agents and/or suspending agents. Liquid formulations may also be prepared by the reconstitution of a solid, for example, from a sachet.

The compounds of formula (1), their pharmaceutically acceptable salts and/or derived forms may also be used in fast-dissolving, fast-disintegrating dosage forms such as those described in Expert Opinion in Therapeutic Patents, 11 (6), 981-986 by Liang and Chen (2001).

The composition of a typical tablet in accordance with the invention may comprise:

| Ingredient | % w/w |
|---|---|
| Compound of formula (1) | 10.00* |
| Microcrystalline cellulose | 64.12 |
| Lactose | 21.38 |
| Croscarmellose sodium | 3.00 |
| Magnesium stearate | 1.50 |

| | |
|---|---|
| * Quantity adjusted in accordance with drug activity. | |

A typical tablet may be prepared using standard processes known to a formulation chemist, for example, by direct compression, granulation (dry, wet, or melt), melt congealing, or extrusion. The tablet formulation may comprise one or more layers and may be coated or uncoated.

Examples of excipients suitable for oral administration include carriers, for example, cellulose, calcium carbonate, dibasic calcium phosphate, mannitol and sodium citrate, granulation binders, for example, polyvinylpyrrolidine, hydroxypropylcellulose, hydroxypropylmethylcellulose and gelatin, disintegrants, for example, sodium starch glycolate and silicates, lubricating agents, for example, magnesium stearate and stearic acid, wetting agents, for example, sodium lauryl sulphate, preservatives, anti-oxidants, flavours and colourants.

Solid formulations for oral administration may be formulated to be immediate and/or modified release. Modified release formulations include delayed-, sustained-, pulsed-, controlled dual-, targeted and programmed release. Details of suitable modified release technologies such as high energy dispersions, osmotic and coated particles are to be found in Verma *et al,* Pharmaceutical Technology On-line, 25(2), 1-14 (2001). Other modified release formulations are described in US Patent No. 6,106,864.

### PARENTERAL ADMINISTRATION

The compounds of formula (1), their pharmaceutically acceptable salts and/or derived forms may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous.

Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (1), their pharmaceutically acceptable salts and/or derived forms used in the preparation of parenteral solutions may be increased by suitable processing, for example, the use of high energy spray-dried dispersions (see WO 01/47495) and/or by the use of appropriate formulation techniques, such as the use of solubility-enhancing agents.

Formulations for parenteral administration may be formulated to be immediate and/or modified release. Modified release formulations include delayed-, sustained-, pulsed-, controlled dual-, targeted and programmed release.

### TOPICAL ADMINISTRATION

The compounds of formula (1), their pharmaceutically acceptable salts and/or derived forms may also be administered topically to the skin or mucosa, either dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin and propylene glycol. Penetration enhancers may be incorporated - see, for example, J Pharm Sci, 88 (10), 955-958 by Finnin and Morgan (October 1999).

Other means of topical administration include delivery by iontophoresis, electroporation, phonophoresis, sonophoresis and needle-free or microneedle injection.

Formulations for topical administration may be formulated to be immediate and/or modified release. Modified release formulations include delayed-, sustained-, pulsed-, controlled dual-, targeted and programmed release. Thus compounds of the invention may be formulated in a more solid form for administration as an implanted depot providing long-term release of the active compound.

### INHALED/INTRANASAL ADMINISTRATION

The compounds of formula (1), their pharmaceutically acceptable salts and/or derived forms can also be administered intranasally or by inhalation, typically in the form of a dry powder (either alone, as a mixture, for example, in a dry blend with lactose in anhydrous or monohydrate form, preferably monohydate, mannitol, dextran, glucose, maltose, sorbitol, xylitol, fructose, sucrose or trehalose, or as a mixed component particle, for example, mixed with phospholipids) from a dry powder inhaler or as an aerosol spray from a pressurised container, pump, spray, atomiser (preferably an atomiser using electrohydrodynamics to produce a fine mist), or nebuliser, with or without the use of a suitable propellant, such as dichlorofluoromethane.

The pressurised container, pump, spray, atomizer, or nebuliser contains a solution or suspension of the active compound comprising, for example, ethanol (optionally, aqueous ethanol) or a suitable alternative agent for dispersing, solubilising, or extending release of the active, the propellant(s) as solvent and an optional surfactant, such as sorbitan trioleate or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

A suitable solution formulation for use in an atomiser using electrohydrodynamics to produce a fine mist may contain from 1µg to 20mg of the compound of the invention per actuation and the actuation volume may vary from 1µl to 100µl. A typical formulation may comprise a compound of formula (1), propylene glycol, sterile water, ethanol and sodium chloride. Alternative solvents which may be used instead of propylene glycol include glycerol and polyethylene glycol.

Capsules, blisters and cartridges (made, for example, from gelatin or HPMC) for use in an inhaler or insufflator may be formulated to contain a powder mix of the compound of the invention, a suitable powder base such as lactose or starch and a performance modifier such as *I*-leucine, mannitol, or magnesium stearate.

In the case of dry powder inhalers and aerosols, the dosage unit is determined by means of a valve which delivers a metered amount. Units in accordance with the invention are typically arranged to administer a metered dose or "puff" containing from 0.001 mg to 10mg of the compound of formula (1). The overall daily dose will typically be in the range 0.001mg to 40mg which may be administered in a single dose or, more usually, as divided doses throughout the day.

Formulations for inhaled/intranasal administration may be formulated to be immediate and/or modified release. Modified release formulations include delayed-, sustained-, pulsed-, controlled, dual-, targeted and programmed release.

Flavouring agent such as menthol or levomenthol and/or sweeteners such as saccharin or saccharin sodium can be added to the formulation

### RECTAL/INTRAVAGINAL ADMINISTRATION

The compounds of formula (1), their pharmaceutically acceptable salts and/or derived forms may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled dual-, targeted and programmed release.

### OCULAR/ANDIAL ADMINISTRATION

The compounds of formula (1), their pharmaceutically acceptable salts and/or derived forms may also be administered directly to the eye or ear, typically in the form of drops of a micronised suspension or solution in isotonic, pH-adjusted, sterile saline. Other formulations suitable for ocular and andial administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/andial administration may be formulated to be immediate and/or modified release. Modified release formulations include delayed-, sustained-, pulsed-, controlled dual-, targeted, or programmed release.

### ENABLING TECHNOLOGIES

The compounds of formula (1), their pharmaceutically acceptable salts and/or derived forms may be combined with soluble macromolecular entities such as cyclodextrin or polyethylene glycol-containing polymers to improve their solubility, dissolution rate, taste-masking, bioavailability and/or stability.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, *i.e.* as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

### DOSAGE

For administration to human patients, the total daily dose of the compounds of the invention is typically in the range 0.001mg to 5000mg depending, of course, on the mode of administration. For example, oral administration may require a total daily dose of from 1 mg to 5000mg, while an inhaled dose may only require from 0.001 mg to 40mg. The total daily dose may be administered in single or divided doses.

These dosages are based on an average human subject having a weight of about 65 to 70kg. The physician will readily be able to determine doses for subjects whose weight falls outside this range, such as infants and the elderly.

According to another embodiment of the present invention, the compounds of the formula (1), or pharmaceutically acceptable salts, derived forms or compositions thereof, can also be used as a combination with one or more additional therapeutic agents to be co-administered to a patient to obtain some particularly desired therapeutic end result such as the treatment of pathophysiologically-relevant disease processes including, but not limited to (i) bronchoconstriction, (ii) inflammation, (iii) allergy, (iv) tissue destruction, (v) signs and symptoms such as breathlessness, cough. The second and more additional therapeutic agents may also be a compound of formula (1), or a pharmaceutically acceptable salt, derived forms or compositions thereof, or one or more β2 agonists known in the art. More typically, the second and more therapeutic agents will be selected from a different class of therapeutic agents.

As used herein, the terms "co-administration", "co-administered" and "in combination with", referring to the compounds of formula (1) and one or more other therapeutic agents, is intended to mean, and does refer to and include the following:
- simultaneous administration of such combination of compound(s) of formula (1) and therapeutic agent(s) to a patient in need of treatment, when such components are formulated together into a single dosage form which releases said components at substantially the same time to said patient,
- substantially simultaneous administration of such combination of compound(s) of formula (1) and therapeutic agent(s) to a patient in need of treatment, when such components are formulated apart from each other into separate dosage forms which are taken at substantially the same time by said patient, whereupon said components are released at substantially the same time to said patient,
- sequential administration of such combination compound(s) of formula (1) and therapeutic agent(s) to a patient in need of treatment, when such components are formulated apart from each other into separate dosage forms which are taken at consecutive times by said patient with a significant time interval between each administration, whereupon said components are released at substantially different times to said patient; and
- sequential administration of such combination of compound(s) of formula (1) and therapeutic agent(s) to a patient in need of treatment, when such components are formulated together into a single dosage form which releases said components in a controlled manner whereupon they are concurrently, consecutively, and/or overlapingly administered at the same and/or different times by said patient,
where each part may be administered by either the same or different route.

Suitable examples of other therapeutic agents which may be used in combination with the compound(s) of formula (1), or pharmaceutically acceptable salts, derived forms or compositions thereof, include, but are by no means limited to :
(a) 5-Lipoxygenase (5-LO) inhibitors or 5-lipoxygenase activating protein (FLAP) antagonists,
(b) Leukotriene antagonists (LTRAs) including antagonists of LTB₄, LTC₄, LTD₄, and LTE₄,
(c) Histamine receptor antagonists including H1 and H3 antagonists,
(d) α₁- and α₂-adrenoceptor agonist vasoconstrictor sympathomimetic agents for decongestant use,
(e) muscarinic M3 receptor antagonists or anticholinergic agents,
(f) PDE inhibitors, e.g. PDE3, PDE4 and PDE5 inhibitors,
(g) Theophylline,
(h) Sodium cromoglycate,
(i) COX inhibitors both non-selective and selective COX-1 or COX-2 inhibitors (NSAIDs),
(j) Oral and inhaled glucocorticosteroids,
(k) Monoclonal antibodies active against endogenous inflammatory entities,
(l) Anti-tumor necrosis factor (anti-TNF-α) agents,
(m)Adhesion molecule inhibitors including VLA-4 antagonists,
(n) Kinin-B₁ - and B₂-receptor antagonists,
(o) Immunosuppressive agents,
(p) Inhibitors of matrix metalloproteases (MMPs),
(q) Tachykinin NK₁, NK₂ and NK₃ receptor antagonists,
(r) Elastase inhibitors,
(s) Adenosine A2a receptor agonists,
(t) Inhibitors of urokinase,
(u) Compounds that act on dopamine receptors, e.g. D2 agonists,
(v) Modulators of the NFκβ pathway, e.g. IKK inhibitors,
(w)Agents that can be classed as mucolytics or anti-tussive, and
(x) Antibiotics.

According to the present invention, combination of the compounds of formula (1) with :
- glucocorticosteroids, in particular inhaled glucocorticosteroids with reduced systemic side effects, including prednisone, prednisolone, flunisolide, triamcinolone acetonide, beclomethasone dipropionate, budesonide, fluticasone propionate, ciclesonide, and mometasone furoate, or
- muscarinic M3 receptor antagonists or anticholinergic agents including in particular ipratropium salts, namely bromide, tiotropium salts, namely bromide, oxitropium salts, namely bromide, perenzepine, and telenzepine,
are preferred.

It is to be appreciated that all references herein to treatment include curative, palliative and prophylactic treatment. The description, which follows, concerns the therapeutic applications to which the compounds of formula (1) may be put.

The compounds of formula (1) have the ability to interact with the β2 receptor and thereby have a wide range of therapeutic applications, as described further below, because of the essential role which the β2 receptor plays in the physiology of all mammals.

Therefore, a further aspect of the present invention relates to the compounds of formula (1), or pharmaceutically acceptable salts, derived forms or compositions thereof, for use in the treatment of diseases, disorders, and conditions in which the β2 receptor is involved. More specifically, the present invention also concerns the compounds of formula (1), or pharmaceutically acceptable salts, derived forms or compositions thereof, for use in the treatment of diseases, disorders, and conditions selected from the group consisting of :
- asthma of whatever type, etiology, or pathogenesis, in particular asthma that is a member selected from the group consisting of atopic asthma, non-atopic asthma, allergic asthma, atopic bronchial IgE-mediated asthma, bronchial asthma, essential asthma, true asthma, intrinsic asthma caused by pathophysiologic disturbances, extrinsic asthma caused by environmental factors, essential asthma of unknown or inapparent cause, non-atopic asthma, bronchitic asthma, emphysematous asthma, exercise-induced asthma, allergen induced asthma, cold air induced asthma, occupational asthma, infective asthma caused by bacterial, fungal, protozoal, or viral infection, non-allergic asthma, incipient asthma, wheezy infant syndrome and bronchiolytis,
- chronic or acute bronchoconstriction, chronic bronchitis, small airways obstruction, and emphysema,
- obstructive or inflammatory airways diseases of whatever type, etiology, or pathogenesis, in particular an obstructive or inflammatory airways disease that is a member selected from the group consisting of chronic eosinophilic pneumonia, chronic obstructive pulmonary disease (COPD), COPD that includes chronic bronchitis, pulmonary emphysema or dyspnea associated or not associated with COPD, COPD that is characterized by irreversible, progressive airways obstruction, adult respiratory distress syndrome (ARDS), exacerbation of airways hyper-reactivity consequent to other drug therapy and airways disease that is associated with pulmonary hypertension,
- bronchitis of whatever type, etiology, or pathogenesis, in particular bronchitis that is a member selected from the group consisting of acute bronchitis, acute laryngotracheal bronchitis, arachidic bronchitis, catarrhal bronchitis, croupus bronchitis, dry bronchitis, infectious asthmatic bronchitis, productive bronchitis, staphylococcus or streptococcal bronchitis and vesicular bronchitis,
- bronchiectasis of whatever type, etiology, or pathogenesis, in particular bronchiectasis that is a member selected from the group consisting of cylindric bronchiectasis, sacculated bronchiectasis, fusiform bronchiectasis, capillary bronchiectasis, cystic bronchiectasis, dry bronchiectasis and follicular bronchiectasis.

A still further aspect of the present invention also relates to the use of the compounds of formula (1), or pharmaceutically acceptable salts, derived forms or compositions thereof, for the manufacture of a drug having a β2 agonist activity. In particular, the present inventions concerns the use of the compounds of formula (1), or pharmaceutically acceptable salts, derived forms or compositions thereof, for the manufacture of a drug for the treatment of β2-mediated diseases and/or conditions, in particular the diseases and/or conditions listed above.

As a consequence, the present invention provides a particularly interesting method of treatment of a mammal, including a human being, including treating said mammal with an effective amount of a compound of formula (1), or a pharmaceutically acceptable salt, derived form or composition thereof. More precisely, the present invention provides a particularly interesting method of treatment of a mammal, including a human being, to treat a β2-mediated diseases and/or conditions, in particular the diseases and/or conditions listed above, including treating said mammal with an effective amount of a compound of formula (1), its pharmaceutically acceptable salts and/or derived forms.

The following examples illustrate the preparation of the compounds of the formula (1):

### Example 1: N-(2,6-Dimethoxybenzyl)-2-(3-{(2R)-2-[(2R)-2-hydroxy-2-(4-hydroxy-3-hydroxymethyl-phenyl}-ethylamino]-propyl)-phenyl)-acetamide

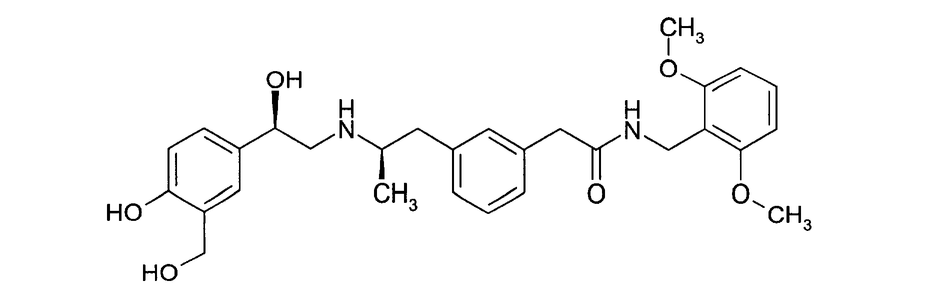

A solution of preparation 1 (125mg, 0.2mmol) in methanol (6ml) was treated with acetic acid (6ml) and ammonium fluoride (74mg, 2.0mmol) and the reaction heated to 40°C for 16 hours. The solvent was removed *in vacuo* and the residue purified by flash column chromatography on silica gel eluting with dichloromethane:methanol:880 ammonia (95:5:0.5 changing to 93:7:0.7, by volume) to give the title compound as a colourless solid after trituration with diethyl ether (59mg).
¹H NMR (400MHz, CD₃OD): δ = 7.24-7.15 (3H, m), 7.09-7.07 (1H, m), 7.02-6.97 (3H, m), 6.70-6.68 (1H, d), 6.61-6.59 (2H, d), 4.61 (2H, s), 4.61-4.58 (1H, m), 4.41 (2H, s), 3.75 (6H, s), 3.43 (2H, s), 2.92-2.84 (2H, m), 2.71-2.67 (2H, m), 2.57-2.52 (1H, dd), 1.05-1.03 (3H, d) ppm.
LRMS (electrospray) : m/z [M+H]⁺ 509, [M-H]⁻ 507.
CHN analysis: found C 67.00%, H 7.48%, N 5.11%; C₂₉H₃₆N₂O₆+0.2Et₂O+ 0.6H₂O requires C 67.00%, H 7.40%, N 5.24%.

### Example 2: N-(2-Ethoxybenzyl)-2-(3-{(2R)-2-[(2R)-2-hydroxy-2-(4-hydroxy-3-hydroxymethyl-phenyl)-ethylamino]-propyl}-phenyl)-acetamide

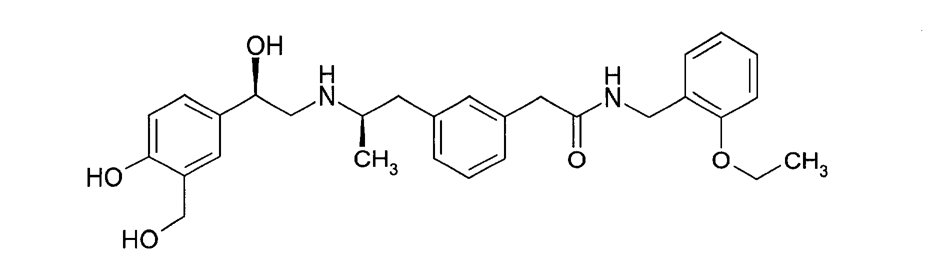

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-*N*-(2-ethoxy benzyl)-acetamide (Preparation 2) according to the method for example 1 to give the title compound as a colourless foam.
¹H NMR (400MHz, CD₃OD): δ = 7.22-7.18 (3H, m), 7.13-7.11 (2H, d), 7.07 (1H, s), 7.03-7.01 (2H, d), 6.90-6.88 (1H, d), 6.85-6.81 (1H, t), 6.71-6.69 (1H, d), 4.65-4.59 (1H, m), 4.62 (2H, s), 4.35 (2H, s), 4.03-3.98 (2H, q), 3.50 (2H, s), 2.99-2.85 (2H, m), 2.77-2.70 (2H, m), 2.59-2.54 (1H, dd), 1.34-1.31 (3H, t), 1.07-1.05 (3H, d) ppm.
LRMS (electrospray) : m/z [M+H]⁺ 493, [M-H]⁻ 491.
CHN analysis : found C 69.25%, H 7.59%, N 5.31%; C₂₉H₃₆N₂O₅+0.15Et₂O+ 0.55H₂O requires C 69.22%, H 7.57%, N 5.45%.

### Example 3: N-(2-Hydroxybenzyl)-2-(3-{(2R)-2-[(2R)-2-hydroxy-2-(4-hydroxy-3-hydroxymethyl-phenyl)-ethylamino]-propyl}-phenyl)-acetamide

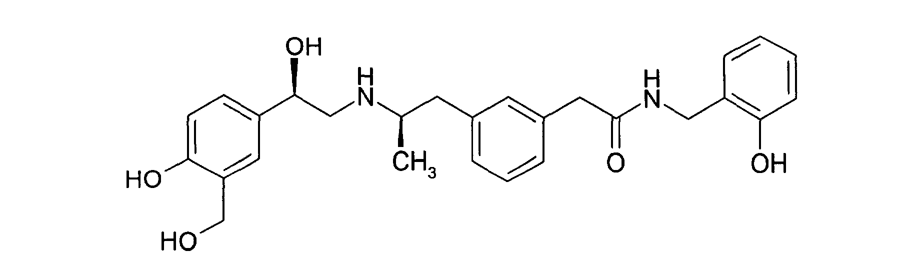

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-*N*-(2-hydroxy benzyl)-acetamide (Preparation 3) according to the method for example 1, using water instead of acetic acid as the co-solvent, to give the title compound as a colourless foam.
¹H NMR (400MHz, CD₃OD): δ = 7.23 (1H, s), 7.19-7.17 (1H, d), 7.12-7.00 (6H, m), 6.77-6.73 (2H, t), 6.71-6.69 (1H, d), 4.65-4.60 (1H, m), 4.62 (2H, s), 4.32 (2H, s), 3.50 (2H, s), 3.00-2.86 (2H, m), 2.75-2.70 (2H, m), 2.60-2.55 (1H, m), 1.07-1.06 (3H, d) ppm.
LRMS (electrospray) : m/z [M+H]⁺ 465, [M+Na]⁺ 487, [M-H]⁻ 463.
CHN analysis : found C 67.46%, H 7.03%, N 5.66%; C₂₇H₃₂N₂O₅+0.9H₂O requires C 67.45%, H 7.09%, N 5.83%.

### Example 4: 2-(3-{(2R)-2-[(2R)-2-Hydroxy-2-(4-hydroxy-3-hydroxymethyl-phenyl)-ethylamino]-propyl}-phenyl)-N-indan-2-yl-acetamide

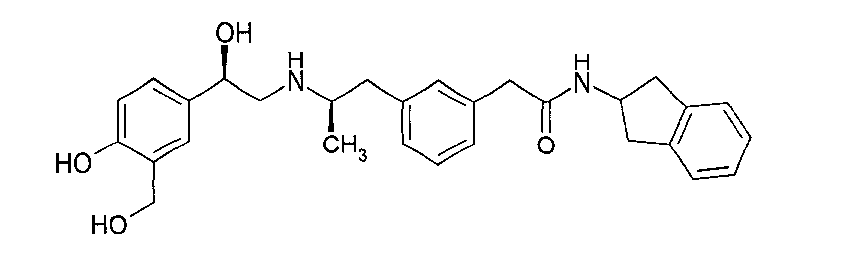

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-*N*-indan-2-yl-acetamide (Preparation 4) according to the method for example 3 to give the title compound as a colourless foam.
¹H NMR (400MHz, CD₃OD): δ = 7.22-7.16 (4H, m), 7.14-7.09 (3H, m), 7.05 (1H, s), 7.03-6.99 (2H, t), 6.70-6.68 (1H, d), 4.63-4.60 (1H, t), 4.61 (2H, s), 4.57-4.53 (1H, m), 3.43 (2H, s), 3.25-3.20 (2H, dd), 2.98-2.93 (1H, q), 2.90-2.85 (1H, dd), 2.84-2.79 (2H, dd), 2.74-2.69 (2H, m), 2.60-2.55 (1H, dd), 1.08-1.07 (3H, d) ppm.
LRMS (electrospray) : m/z [M+H]⁺ 475, [M-H]⁻ 473.
CHN analysis : found C 72.29%, H 7.28%, N 5.79%; C₂₉H₃₄N₂O₄+0.4H₂O
requires C 72.29%, H 7.28%, N 5.81%.

### Example 5: N-(3,4-Dichlorobenzyl)-2-(3-{(2R)-2-[(2R)-2-hydroxy-2-(4-hydroxy-3-hydroxymethyl-phenyl)-ethylamino]-propyl}-phenyl)-acetamide

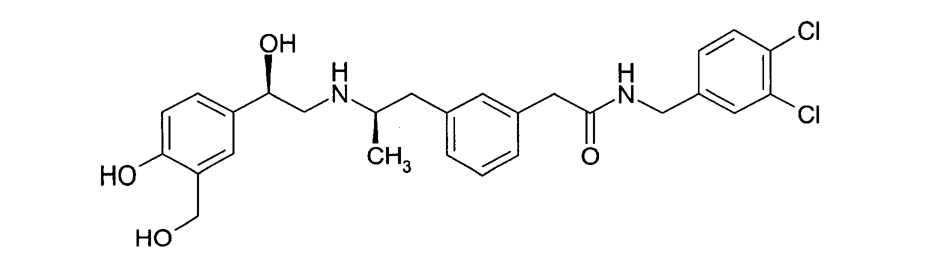

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-*N*-(3,4-dichloro benzyl)-acetamide (Preparation 5) according to the method for example 3 to give the title compound as a colourless foam.
¹H NMR (400MHz, CD₃OD): δ = 7.41-7.38 (1H, d), 7.33 (1H, d), 7.21-7.17 (2H, m), 7.13-7.11 (2H, d), 7.06 (1H, s), 7.01-6.99 (2H, m), 6.69-6.67 (1H, d), 4.60 (2H, s), 4.60-4.57 (1H, m), 4.30 (2H, s), 3.50 (2H, s), 2.96-2.91 (1H, m), 2.88-2.83 (1H, dd), 2.73-2.68 (2H, m), 2.60-2.57 (1H, dd), 1.06-1.05 (3H, d) ppm.
LRMS (electrospray) : m/z [M+H]⁺ 517/519, [M-H]⁻ 515/517.
CHN analysis : found C 61.95%, H 5.93%, N 5.37%; C₂₇H₃₀CI₂N₂O₄+0.35H₂O requires C 61.92%, H 5.91%, N 5.35%.

### Example 6: N-[4-(aminosulfonyl)benzyl]-2-(3-({2R)-2-[(2R)-2-hydroxy-2-(4-hydroxy-3-hydroxymethyl-phenyl)-ethylamino]-propyl}-phenyl)-acetamide

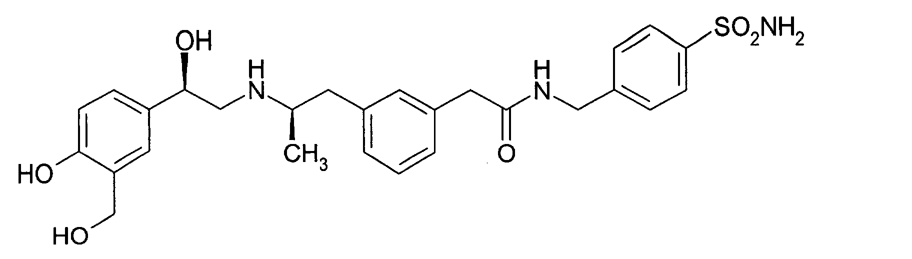

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-*N*-[4-(aminosulfonyl) benzyl]-acetamide (Preparation 6) according to the method for example 3 to give the title compound as a colourless foam.
¹H NMR (400MHz, CD₃OD): δ = 7.82-7.79 (2H, d), 7.38-7.36 (2H, d), 7.25-7.13 (3H, m), 7.09 (1H, s), 7.05-7.03 (2H, d), 6.72-6.70 (1H, d), 4.65-4.62 (1H, m), 4.62 (2H, s), 4.42 (2H, s), 3.53 (2H, s), 3.04-2.98 (1H, m), 2.93-2.86 (1H, m), 2.80-2.75 (2H, m), 2.63-2.57 (1H, m), 1.09-1.08 (3H, d) ppm.
LRMS (electrospray): m/z [M+H]⁺ 528, [M+Na]⁺ 550, [M-H]⁻ 526.
CHN analysis : found C 59.44%, H 6.44%, N 7.65%; C₂₇H₃₃N₃O₆S+1.0H₂O requires C 59.43%, H 6.47%, N 7.70%.

### Example 7: 2-(3-{(2R}-2-[(2R}-2-Hydroxy-2-(4-hydroxy-3-hydroxymethyl-phenyl)-ethylamino]-propyl}-phenyl)-N-(pyridin-2-ylmethyl)-acetamide

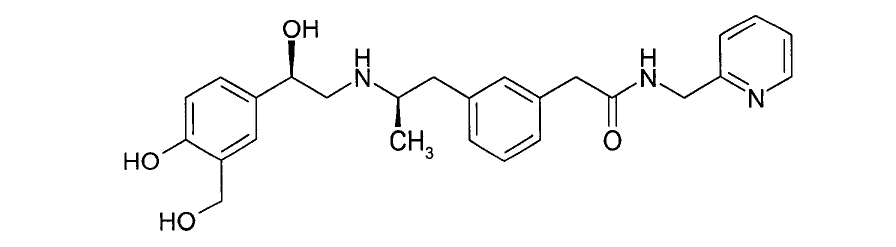

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-*N*-(pyridin-2-ylmethyl)-acetamide (Preparation 7) according to the method for example 3 to give the title compound as a colourless foam.
¹H NMR (400MHz, CD₃OD): δ = 8.44-8.42 (1H, d), 7.75-7.71 (1H, t), 7.28-7.14 (5H, m), 7.11 (1H, s), 7.04-7.01 (2H, t), 6.70-6.68 (1H, d), 4.63-4.60 (1H, m), 4.62 (2H, s), 4.47 (2H, s), 3.57 (2H, s), 3.03-2.95 (1H, m), 2.91-2.86 (1H, dd), 2.77-2.72 (2H, m), 2.64-2.59 (1H, dd), 1.11-1.09 (3H, d) ppm.
LRMS (electrospray) : m/z [M+H]⁺ 450, [M+Na]⁺ 472, [M-H]⁻ 448.
CHN analysis : found C 67.06%, H 6.94%, N 8.96%; C₂₆H₃₁N₃O₄+0.9H₂O requires C 67.05%, H 7.10%, N 9.02%.

### Example 8: 4-{(2-(3-{(2R)-2-[(2R)-2-Hydroxy-2-(4-hydroxy-3-hydroxymethyl-phenyl)-ethylamino]-propyl}-phenyl)-acetylamino)-methyl}-benzamide

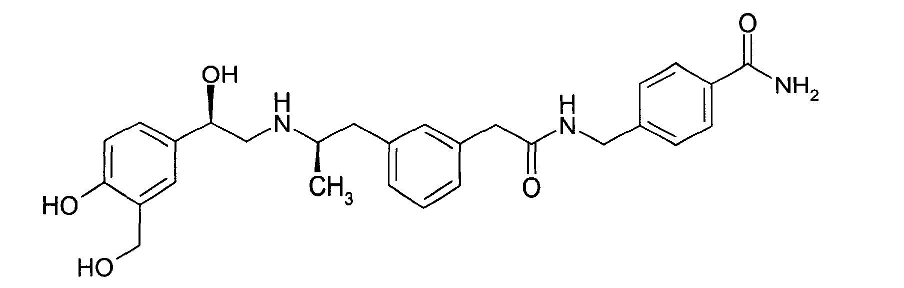

Prepared from 4-{(2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-acetylamino)-methyl}-benzamide (Preparation 8) according to the method for example 3 to give the title compound as a colourless foam.
¹H NMR (400MHz, CD₃OD): δ = 7.80-7.78 (2H, d), 7.31-7.29 (2H, d), 7.21-7.12 (3H, m), 7.09 (1H, s), 7.03-7.01 (2H, d), 6.70-6.68 (1H, d), 4.61-4.59 (1H, m), 4.61 (2H, s), 4.40 (2H, s), 3.52 (2H, s), 2.98-2.94 (1H, m), 2.90-2.85 (1H, m), 2.74-2.70 (2H, m), 2.61-2.56 (1H, dd), 1.08-1.06 (3H, d) ppm.
LRMS (electrospray) : m/z [M+H]⁺ 492, [M+Na]⁺ 514, [M-H]⁻ 490.
CHN analysis : found C 65.27%, H 6.73%, N 8.20%; C₂₈H₃₃N₃O₅+1.3H₂O requires C 65.30%, H 6.97%, N 8.16%.

### Example 9: N-(3,4-Dimethoxybenzyl)-2-(3-{(2R)-2-[(2R)-2-hydroxy-2-(4-hydroxy-3-hydroxymethyl-phenyl)-ethylamino]-propyl}-phenyl)-acetamide

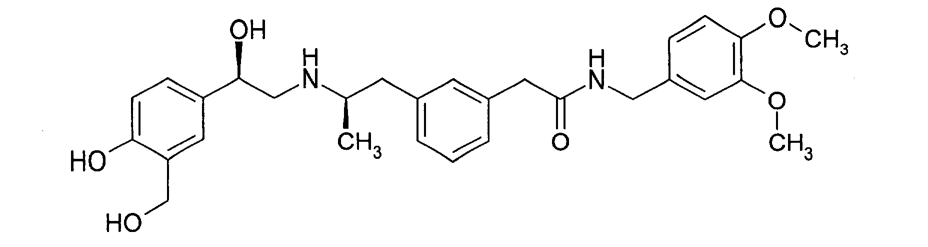

Prepared from 2-(3-{(2*R*)-2-[(2*R*)-2-{[*tert*-Butyl(dimethyl)silyl]oxy}-2-(4-hydroxy -3-hydroxymethyl-phenyl)-ethylamino]-propyl}-phenyl)-*N*-(3,4-dimethoxy benzyl)-acetamide (Preparation 9) according to the method for example 3 to give the title compound as a colourless foam.
¹H NMR (400MHz, CD₃OD): δ = 7.21-7.13 (3H, m), 7.08 (1H, s), 7.02-7.00 (2H, d), 6.86-6.77 (3H, m), 6.70-6.68 (1H, d), 4.65-4.59 (1H, m), 4.61 (2H, s), 4.28 (2H, s), 3.78 (3H, s), 3.71 (3H, s), 3.49 (2H, s), 2.96-2.84 (2H, m), 2.73-2.66 (2H, m), 2.59-2.54 (1H, dd), 1.07-1.05 (3H, d) ppm.
LRMS (electrospray) : m/z [M+H]⁺ 509, [M+Na]⁺ 531, [M-H]⁻ 507.
CHN analysis : found C 66.69%, H 7.44%, N 5.14%;
C₂₉H₃₆N₂O₆+0.2Et₂O+0.75H₂O requires C 66.66%, H 7.41%, N 5.22%.

### Example 10: 2-(3-{(2R)-2-[(2R)-2-Hydroxy-2-(4-hydroxy-3-hydroxymethyl-phenyl)-ethylamino]-propyl}-phenyl)-N-(4-trifluoromethoxybenzyl)-acetamide

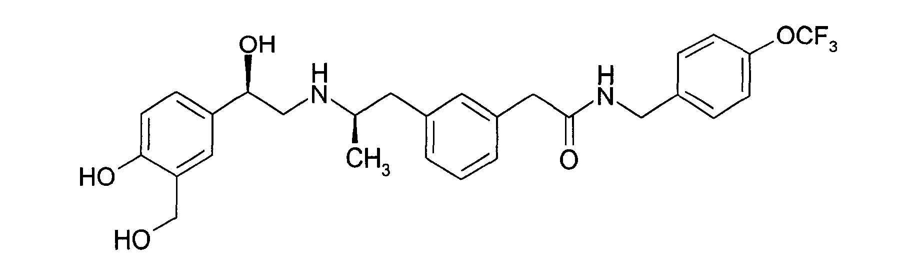

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-*N*-(4-trifluoromethoxybenzyl)-acetamide (Preparation 10) using the method for example 3 to give the title compound as a colourless foam.
¹H NMR (400MHz, CD₃OD): δ = 7.32-7.30 (2H, d), 7.22-7.12 (5H, m), 7.08 (1H, s), 7.03-7.01 (2H, d), 6.70-6.68 (1H, d), 4.62-4.59 (1H, m), 4.61 (2H, s), 4.36 (2H, s), 3.51 (2H, s), 3.00-2.93 (1H, m), 2.90-2.85 (1H, dd), 2.73-2.69 (2H, m), 2.61-2.56 (1H, dd), 1.08-1.06 (3H, d) ppm.
LRMS (electrospray) : m/z [M+H]⁺ 533, [M-H]⁻ 531.
CHN analysis : found C 62.12%, H 5.98%, N 5.15%; C₂₈H₃₁F₃N₂O₅+0.5H₂O requires C 62.10%, H 5.96%, N 5.17%.

### Example 11 : N-(2-Chloro, 6-fluorobenzyl)-2-(3-{(2R)-2-[(2R)-2-hydroxy-2-(4-hydroxy-3-hydroxymethyl-phenyl)-ethylamino]-propyl}-phenyl)-acetamide

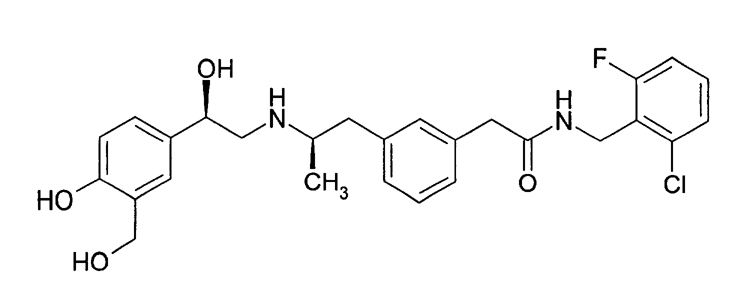

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-*N*-(2-chloro, 6-fluorobenzyl)-acetamide (Preparation 11) using the method for example 3 to give the title compound as a colourless foam.
¹H NMR (400MHz, CD₃OD): δ = 7.33-7.28 (1H, m), 7.25-7.22 (2H, m), 7.18-7.15 (1H, t), 7.09-6.97 (5H, m), 6.70-6.68 (1H, d), 4.63-4.60 (1H, m), 4.61 (2H, s), 4.53 (2H, s), 3.45 (2H, s), 2.97-2.91 (1H, m), 2.90-2.85 (1H, dd), 2.74-2.69 (2H, m), 2.57-2.52 (1H, dd), 1.06-1.05 (3H, d) ppm.
LRMS (electrospray) : m/z [M+H]⁺ 501/503, [M-H]⁻ 499/501.
CHN analysis : found C 62.90%, H 6.06%, N 5.34%; C₂₇H₃₀CIF N₂O₄+0.8H₂O requires C 62.92%, H 6.18%, N 5.44%.

### Example 12: N-(3,4-Dimethylbenzyl)-2-(3-{(2R)-2-[(2R)-2-hydroxy-2-(4-hydroxy-3-hydroxymethyl-phenyl)-ethylamino]-propyl}-phenyl)-acetamide

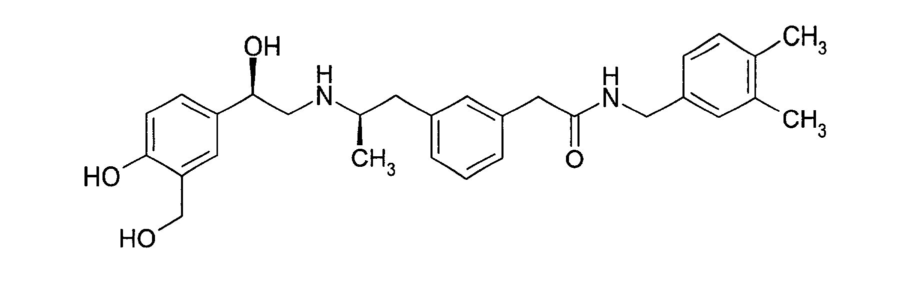

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-*N*-(3,4-dimethylbenzyl)-acetamide (Preparation 12) using the method for example 3 to give the title compound as a colourless foam.
¹H NMR (400MHz, CD₃OD): δ = 7.21-7.12 (3H, m), 7.07 (1H, s), 7.05-7.00 (3H, m), 6.95-6.91 (2H, m), 6.70-6.68 (1H, d), 4.62-4.59 (1H, m), 4.61 (2H, s), 4.26 (2H, s), 3.49 (2H, s), 2.98-2.90 (1H, m), 2.90-2.85 (1H, dd), 2.74-2.69 (2H, m), 2.60-2.55 (1H, dd), 2.20 (3H, s), 2.19 (3H, s), 1.07-1.05 (3H, d) ppm.
LRMS (electrospray) : m/z [M+H]⁺ 477, [M-H]⁻ 475.
CHN analysis : found C 71.69, H 7.70, N 5.72; C₂₉H₃₆N₂O₄+0.5H₂O requires C 71.73%, H 7.68%, N 5.77%.

### Example 13: N-[2-Fluoro-5-(trifluoromethyl)benzyl]-2-(3-{(2R)-2-[(2R)-2-hydroxy-2-(4-hydroxy-3-hydroxymethyl-phenyl)-ethylamino]-propyl}-phenyl)-acetamide

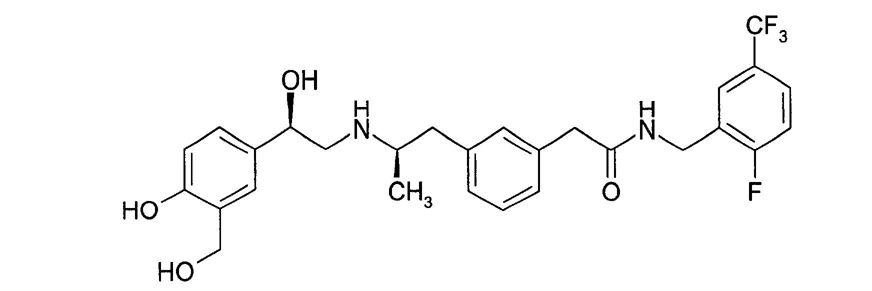

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-N-[2-fluoro-5-(trifluoromethyl)benzyl]-acetamide (Preparation 13) using the method for example 3 to give the title compound as a colourless foam.
¹H NMR (400MHz, CD₃OD): δ = 7.61-7.57 (1H, m), 7.55-7.52 (1H, m), 7.28-7.18 (3H, m), 7.14-7.12 (1H, m), 7.07 (1H, s), 7.03-7.01 (2H, d), 6.70-6.68 (1H, d), 4.63-4.60 (1H, m), 4.61 (2H, s), 4.44 (2H, s), 3.52 (2H, s), 3.00-2.93 (1H, m), 2.91-2.86 (1H, dd), 2.76-2.70 (2H, m), 2.60-2.55 (1H, dd), 1.07-1.06 (3H, d) ppm.
LRMS (electrospray) : m/z [M+H]⁺ 535, [M-H]⁻ 533.
CHN analysis : found C 62.26%, H 5.81%, N 5.09%; C₂₈H₃₀F₄N₂O₄+0.30H₂O requires C 62.28%, H 5.71%, N 5.19%.

### Example 14: N-(2,6-Dichlorobenzyl)-2-(3-{(2R)-2-[(2R)-2-hydroxy-2-(4-hydroxy-3-hydroxymethyl-phenyl)-ethylamino]-propyl}-phenyl)-acetamide

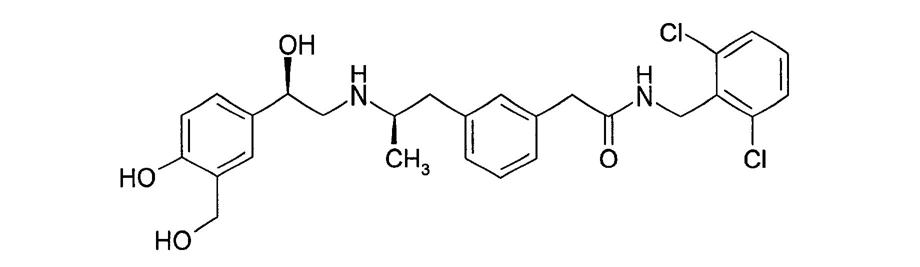

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-*N*-(2,6-dichlorobenzyl)-acetamide (Preparation 14) using the method for example 3 to give the title compound as a colourless foam.
¹H NMR (400MHz, CD₃OD): δ = 7.38-7.36 (2H, d), 7.27-6.97 (7H, m), 6.70-6.68 (1H, d), 4.69-4.55 (5H, m), 3.46 (2H, s), 3.00-2.83 (2H, m), 2.79-2.68 (2H, m), 2.61-2.50 (1H, m), 1.07-1.05 (3H, d) ppm.
LRMS (electrospray) : m/z [M+H]⁺ 517 / 519, [M+Na]⁺ 539 / 541, [M-H]⁻ 515 / 517.
CHN analysis: found C 61.40%, H 6.13%, N 5.15%; C₂₇H₃₀Cl₂N₂O₄+0.6H₂O requires C 61.39%, H 5.95%, N 5.30%.

### Example 15: 2-(3-{(2R)-2-[(2R)-2-hydroxy-2-(4-hydroxy-3-hydroxymethyl-phenyl)-ethylamino]-propyl}-phenyl)-N-(2-phenylethyl)acetamide

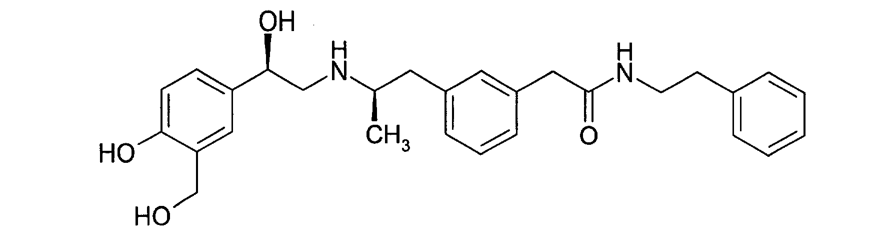

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-*N*-(2-phenylethyl)acetamide (Preparation 15) using the method for example 3 to give the title compound as a colourless gummy solid.
¹H NMR (400MHz, CD₃OD): δ = 7.49-7.30 (8H, m), 7.26-7.22 (3H, m), 6.90-6.88 (1H, d), 4.81-4.78 (3H, m), 3.60-3.54 (4H, m), 3.16-3.00 (2H, m), 2.96-2.87 (4H, m), 2.80-2.75 (1H, dd), 1.28-1.26 (3H, d) ppm.
LRMS (electrospray) : m/z [M+H]⁺ 463, [M+Na]⁺ 485, [M-H]⁻ 461.
CHN analysis : found C 69.89%, H 7.63%, N 5.98%; C₂₈H₃₄N₂O₄+1.30H₂O requires C 69.20%, H 7.59%, N 5.76%.

### Example 16: N-Benzyl-2-(3-{(2R)-2-[(2R)-2-hydroxy-2-(4-hydroxy-3-hydroxymethyl-phenyl)-ethylamino]-propyl}-phenyl)-acetamide

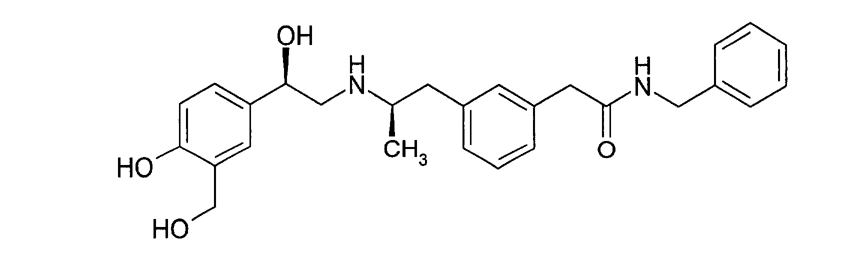

Prepared from *N*-Benzyl-2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-acetamide (Preparation 16) using the method for example 3 to give the title compound as a colourless gum.
¹H NMR (400MHz, CD₃OD): δ = 7.29-7.18 (7H, m), 7.14-7.12 (1H, m), 7.08 (1H, s), 7.05-6.99 (2H, m), 6.71-6.69 (1H, d), 4.64-4.62 (1H, m), 4.61 (2H, s), 4.34 (2H, s), 3.51 (2H, s), 3.02-2.93 (1H, m), 2.91-2.86 (1H, m), 2.76-2.72 (2H, dd), 2.61-2.56 (1H, dd), 1.08-1.07 (3H, d) ppm.
LRMS (electrospray) : m/z [M+H]⁺ 449, [M+Na]⁺ 471, [M-H]⁻ 447.
CHN analysis : found C 69.02%, H 7.24%, N 5.95%; C₂₇H₃₂N₂O₄+1.15H₂O requires C 69.11%, H 7.37%, N 5.97%.

### Example 17: N-(3,5-Dichlorobenzyl)-2-(3-{(2R)-2-[(2R)-2-hydroxy-2-(4-hydroxy-3-hydroxymethyl-phenyl)-ethylamino]-propyl}-phenyl)-acetamide

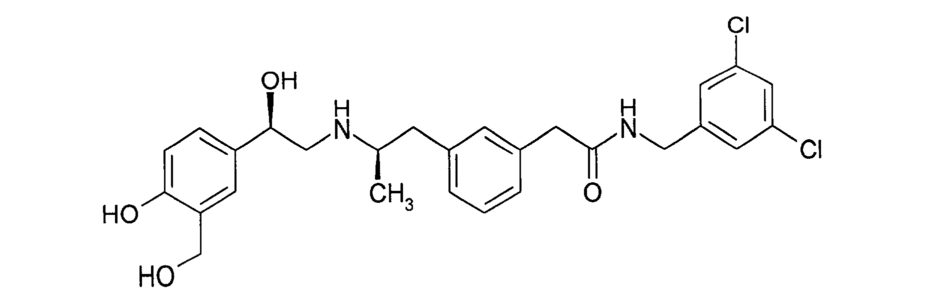

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-*N*-(3,5-dichlorobenzyl)-acetamide (Preparation 17) using the method for example 3 to give the title compound as a colourless foam.
¹H NMR (400MHz, CD₃OD): δ = 7.28-7.13 (6H, m), 7.08 (1H, s), 7.04-7.01 (2H, m), 6.71-6.69 (1H, d), 4.62 (2H, s), 4.62-4.59 (1H, m), 4.31 (2H, s), 3.52 (2H, s), 3.02-2.92 (1H,m), 2.90-2.85 (1H, m), 2.77-2.69 (2H, m), 2.62-2.57 (1H, dd), 1.08-1.06 (3H, d) ppm.
LRMS (electrospray): m/z [M+H]⁺ 517 / 519, [M+Na]⁺ 539 / 541, [M-H]⁻ 515 / 517.
CHN analysis : found C 60.61%, H 5.86%, N 5.14%; C₂₇H₃₀Cl₂N₂O₄+0.95H₂O requires C 60.67%, H 6.01%, N 5.24%.

### Example 18: N-(4-Chlorobenzyl)-2-(3-{(2R)-2-[(2R)-2-hydroxy-2-(4-hydroxy-3-hydroxymethyl-phenyl)-ethylamino]-propyl}-phenyl)-acetamide

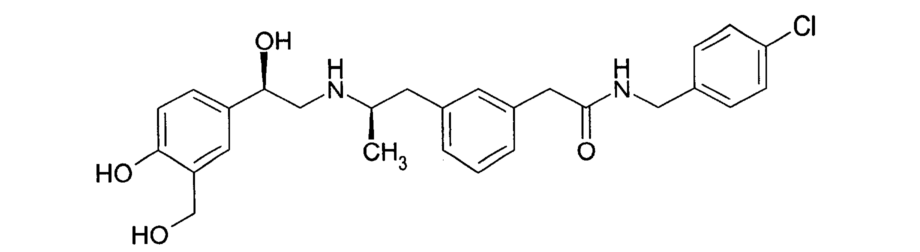

Prepared from 2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-*N*-(4-chlorobenzyl)-acetamide (Preparation 18) using the method for example 3 to give the title compound as a colourless foam.
¹H NMR (400MHz, CD₃OD): δ = 7.28-7.18 (6H, m), 7.13-7.11 (1H, m), 7.06 (1H, s), 7.02-7.00 (2H, d), 6.70-6.68 (1H, d), 4.61 (2H, s), 4.61-4.58 (1H, m), 4.32 (2H, s), 3.50 (2H, s), 2.95-2.90 (1H, m), 2.89-2.84 (1H, dd), 2.72-2.67 (2H, m), 2.60-2.55 (1H, dd), 1.07-1.05 (3H, d) ppm.
LRMS (electrospray) : m/z [M+H]⁺ 483 / 485, [M+Na]⁺ 505 / 507, [M-H]⁻ 481 / 483.
CHN analysis: found C 64.92%, H 6.46%, N 5.61%; C₂₇H₃₁ClN₂O₄+0.9H₂O requires C 64.96%, H 6.62%, N 5.61%.

### Example 19: 4-{([2-(3-{(2R)-2-[(2R)-2-hydroxy-2-(4-hydroxy-3-hydroxymethyl-phenyl)-ethylamino]-propyl}-phenyl)-acetyl]-amino)-methyl}-benzoic acid

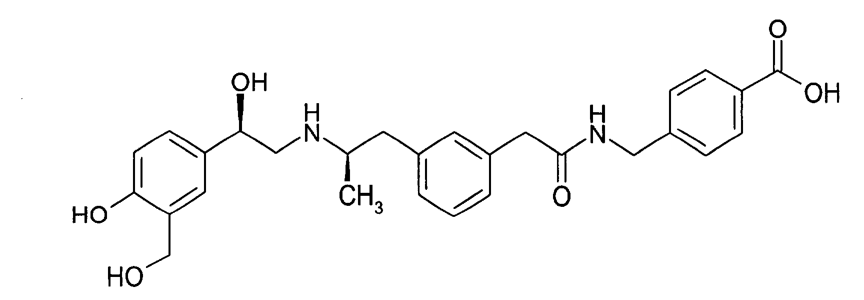

Prepared from 4-{([2-(3-{(2*R*)-2-[(2*R*)-2-{[*tert*-Butyl(dimethyl)silyl]oxy}-2-(4-hydroxy-3-hydroxymethyl-phenyl)-ethylamino]-propyl}-phenyl)-acetyl]-amino)-methyl}-benzoic acid (Preparation 19) using the method for example 3 to give the title compound as a colourless foam.
¹H NMR (400MHz, CD₃OD): δ = 7.86-7.84 (2H, d), 7.33 (1H, s), 7.28-7.09 (7H, m), 6.78-6.76 (1H, d), 4.80 (1H, m, partially obscured by solvent), 4.65 (2H, s), 4.38 (2H, s), 3.54 (2H, s), 3.43-3.36 (1H, m), 3.13-3.05 (3H, m), 2.71-2.65 (1H, m), 1.16-1.14 (3H, d) ppm.
LRMS (electrospray) : m/z [M+H]⁺ 493, [M+Na]⁺ 515, [M-H]⁻ 491.

### Example 20: N-(2,6-Dichlorobenzyl)-3-(3-{(2R)-2-[(2R)-2-hydroxy-2-(4-hydroxy-3-hydroxymethyl-phenyl)-ethylamino]-propyl}-phenyl)-propanamide

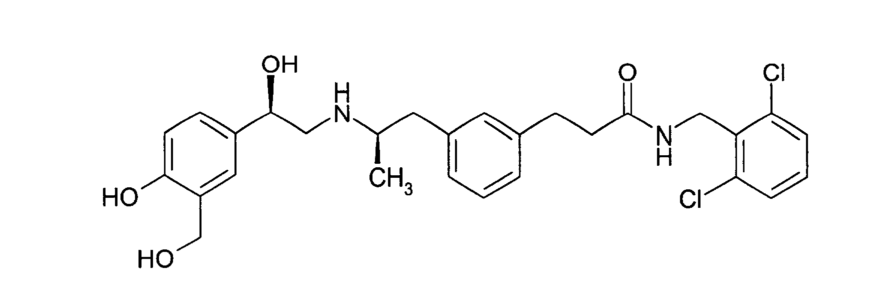

Prepared from 2-(3-{(2*R*)-2-[(2*R*)-2-{[*tert*-butyl(dimethyl)silyl]oxy}-2-(4-hydroxy -3-hydroxymethyl-phenyl)-ethylamino]-propyl}-phenyl)-*N*-(2,6-dichlorobenzyl)-propanamide (Preparation 21) using the method for example 3 to give the title compounds as a colourless foam.
¹H NMR (400MHz, CD₃OD): δ = 7.39-7.37 (2H, d), 7.28-7.22 (2H, m), 7.15-7.11 (1H, t), 7.03-7.01 (2H, d), 6.98 (1H, s), 6.95-6.93 (1H, d), 6.71-6.69 (1H, d), 4.66-4.58 (5H, m), 3.05-2.09 (1H, m), 2.94-2.84 (3H, m), 2.79-2.71 (2H, m), 2.60-2.54 (1H, dd), 2.48-2.41 (2H, t), 1.09-1.08 (3H, d) ppm.
LRMS (electrospray) : m/z [M+H]⁺ 531 / 533, [M+Na]⁺ 553 / 555, [M-H]⁻ 529 / 531.
CHN analysis: found C 60.34%, H 6.00%, N 4.89%; C₂₈H₃₂CI₂N₂O₄+1.45 H₂O requires C 60.31%, H 6.31%, N 5.02%.

### Preparation 1: 2-(3-{(2R)-2-[(2R)-2-([tert-Butyl(dimethyl)silyl]oxy}-2-(4-hydroxy-3-hydroxymethyl-phenyl)-ethylamino]-propyl}-phenyl)-N-(2,6-dimethoxybenzyl)-acetamide

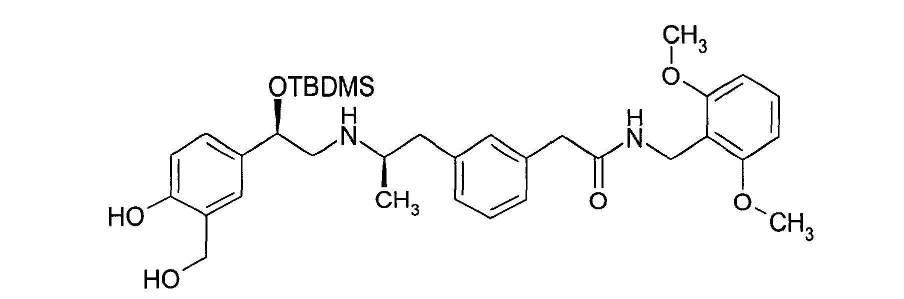

A solution of the acid from preparation 22 (150mg, 0.32mmol), 1-(3-dimethylaminopropyl)-3-ethylcarbodiimide hydrochloride (67mg, 0.35mmol), hydroxybenzotriazole monohydrate (47mg, 0.35 mmol) in *N, N*-dimethylformamide (3 ml) was treated with triethylamine (0.09ml, 0.63 mmol) and 2,6-dimethoxybenzylamine (58mg, 0.35 mmol) and the resulting suspension left to stir at room temperature under a nitrogen atmosphere for 18 hours. The solvent was removed *in vacuo* and the residue partitioned between ethyl acetate (10 ml) and saturated aqueous sodium bicarbonate (10 ml). The organic phase was separated, and the aqueous phase extracted with further ethyl acetate (2 x 10ml). The combined organic extracts were washed with water (5ml), saturated aqueous sodium chloride (5ml), dried (sodium sulphate) and the solvent removed *in vacuo.* The residue was purified by flash column chromatography on silica gel eluting with dichloromethane:methanol: 880 ammonia (95:5:0.5 by volume) to give the title compound as a pale yellow oil (128mg).
¹H NMR (400MHz, CD₃OD): δ = 7.23-7.14 (3H, m), 7.09-7.07 (1H, d), 6.99-6.93 (3H, m), 6.69-6.67 (1H, d), 6.59-6.61 (2H, d), 4.70-4.67 (1H, m), 4.61 (2H, d), 4.41 (2H, s), 3.75 (6H, s), 3.42 (2H, s), 2.89-2.81 (2H, m), 2.67-2.60 (2H, m), 2.53-2.48 (1H, dd), 1.02-1.01 (3H, d), 0.84 (9H, s), 0.00 (3H, s), -0.19 (3H, s) ppm.
LRMS (electrospray) : m/z [M+H]⁺ 623, [M+Na]⁺ 645, [M-H]⁻ 621.

### Preparation 2: 2-(3-{(2R)-2-[(2R)-2-([tert-Butyl(dimethyl)silyl]oxy}-2-(4-hydroxy-3-hydroxymethyl-phenyl)-ethylamino]-propyl}-phenyl)-N-(2-ethoxybenzyl)-acetamide

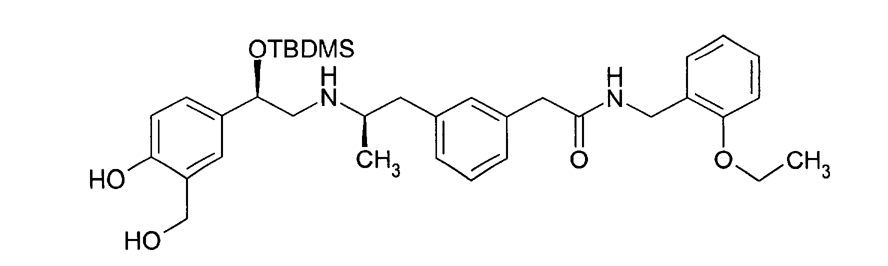

Prepared according to the procedure used for preparation 1 using the acid from preparation 22 and the appropriate amine to give the title compound as a colourless solid.
¹H NMR (400MHz, CD₃OD): δ = 7.21-7.17 (3H, m), 7.14-7.12 (2H, d), 7.04 (1H, s), 7.00-6.98 (2H, d), 6.91-6.89 (1H, d), 6.85-6.81 (1H, t), 6.70-6.68 (1H, d), 4.72-4.68 (1H, t), 4.62-4.61 (2H, d), 4.35 (2H, s), 4.04-3.98 (2H, q), 3.50 (2H, s), 2.94-2.84 (2H, m), 2.72-2.62 (2H, m), 2.56-2.51 (1H, dd), 1.34-1.30 (3H, t), 1.04-1.02 (3H, d), 0.84 (9H, s), 0.00 (3H, s), -0.19 (3H, s) ppm.
LRMS (electrospray) : m/z [M+H]⁺ 607, [M+Na]⁺ 629, [M-H]⁻ 605.

### Preparation 3: 2-(3-{(2R)-2-[(2R)-2-{[tert-Butyl(dimethyl)silyl]oxy}-2-(4-hydroxy-3-hydroxymethyl-phenyl)-ethylamino]-propyl}-phenyl)-N-(2-hydroxybenzyl)-acetamide

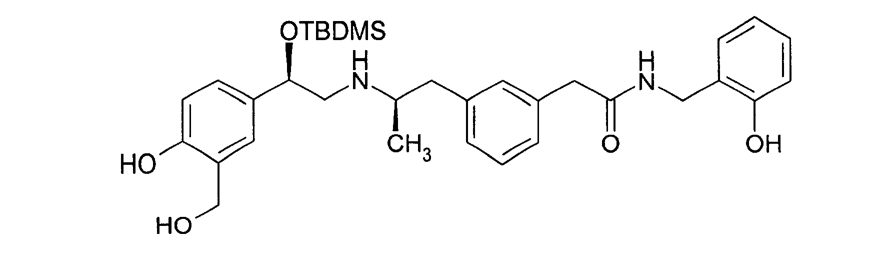

Prepared according to the procedure used for preparation 1 using the acid from preparation 22 and the appropriate amine, substituting dichloromethane as the reaction solvent to give the title compound as a colourless solid.
¹H NMR (400MHz, CD₃OD): δ = 7.19-7.02 (6H, m), 6.99-6.95 (2H, m), 6.75-6.70 (2H, m), 6.68-6.66 (1H, d), 4.71-4.67 (1H, t), 4.61 (2H, d), 4.32 (2H, s), 3.49 (2H, s), 2.91-2.84 (2H, m), 2.70-2.61 (2H, m), 2.55-2.50 (1H, dd), 1.04-1.03 (3H, d), 0.84 (9H, s), 0.01 (3H, s), -0.18 (3H, s) ppm.
LRMS (electrospray) : m/z [M+H]⁺ 579, [M+Na]⁺ 601, [M-H]⁻ 577.

### Preparation 4: 2-(3-{(2R)-2-[(2R)-2-{[tert-Butyl(dimethyl)silyl]oxy}-2-(4-hydroxy-3-hydroxymethyl-phenyl)-ethylamino]-propyl}-phenyl)-N-indan-2-yl-acetamide

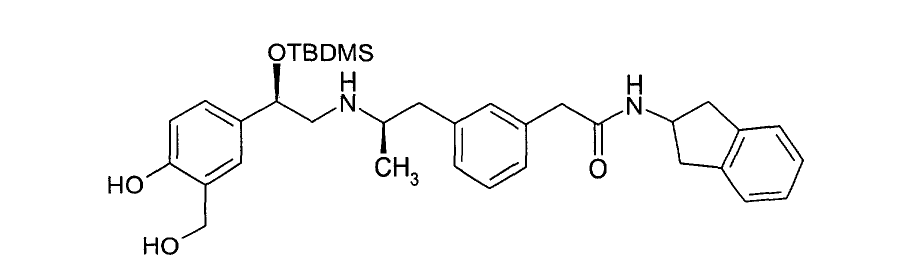

Prepared according to the procedure used for preparation 3 using the acid from preparation 22 and the appropriate amine to give the title compound as a colourless solid.
¹H NMR (400MHz, CD₃OD): δ = 7.20-7.09 (7H, m), 7.02 (1H, s), 6.99-6.96 (2H, t), 6.69-6.67 (1H, d), 4.71-4.68 (1H, m), 4.61-4.60 (2H, d), 4.56-4.52 (1H, t) 3.42 (2H, s), 3.26-3.20 (2H, dd), 2.92-2.79 (4H, m), 2.69-2.62 (2H, m), 2.56-2.51 (1H, m), 1.05-1.03 (3H, d), 0.83 (9H, s), 0.00 (3H, s), -0.20 (3H, s) ppm.
LRMS (electrospray) : m/z [M+H]⁺ 589, [M+Na]⁺ 611, [M-H]⁻ 587.

### Preparation 5: 2-(3-{(2R)-2-[(2R)-2-{[tert-Butyl(dimethyl)silyl]oxy}-2-(4-hydroxy-3-hydroxymethyl-phenyl)-ethylamino]-propyl}-phenyl)-N-(3,4-dichlorobenzyl)-acetamide

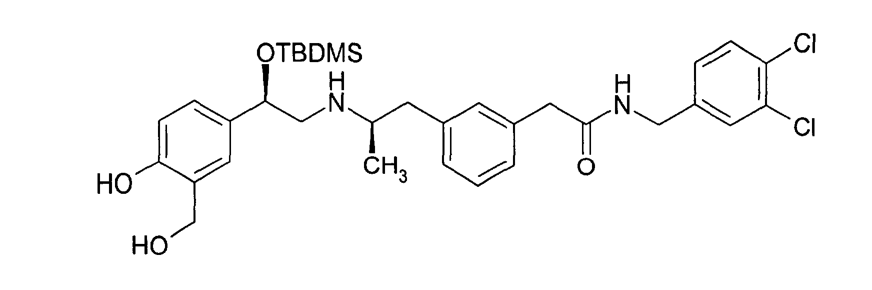

Prepared according to the procedure used for preparation 1 using the acid from preparation 22 and the appropriate amine substituting dichloromethane instead of ethyl acetate as the extraction solvent to give the title compound as a colourless solid.
¹H NMR (400MHz, CD₃OD): δ = 7.41-7.39 (1H, d), 7.34 (1H, s), 7.20-7.16 (2H, m), 7.14-7.10 (2H, t), 7.03 (1H, s), 7.00-6.96 (2H, d), 6.68-6.66 (1H, d), 4.70-4.67 (1H, t), 4.61-4.60 (2H, d), 4.30 (2H, s), 3.49 (2H, s), 2.92-2.84 (2H, m), 2.70-2.60 (2H, m), 2.56-2.51 (1H, dd), 1.03-1.01 (3H, d), 0.82 (9H, s), -0.01 (3H, s), -0.20 (3H, s) ppm.
LRMS (electrospray) : m/z [M+H]⁺ 631 / 633, [M+Na]⁺ 653 / 655, [M-H]⁻ 629 / 631.

### Preparation 6: 2-(3-{(2R)-2-[(2R)-2-{[tert-Butyl(dimethyl)silyl]oxy}-2-(4-hydroxy-3-hydroxymethyl-phenyl)-ethylamino]-propyl}-phenyl)-N-[4-(aminosulfonyl)benzyl]-acetamide

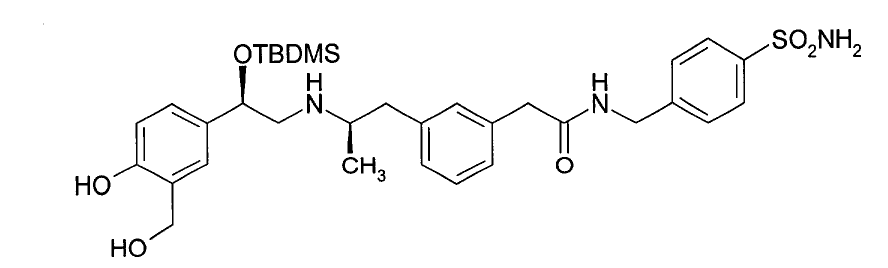

Prepared according to the procedure used for preparation 5 using the acid from preparation 22 and the appropriate amine to give the title compound as a colourless gum.
¹H NMR (400MHz, CD₃OD): δ = 7.82-7.79 (2H, d), 7.38-7.36 (2H, d), 7.22-7.13 (3H, m), 7.05 (1H, s), 7.01-6.99 (2H, d), 6.70-6.67 (1H, d), 4.72-4.69 (1H, t), 4.62-4.61 (2H, d), 4.42 (2H, s), 3.51 (2H, s), 2.95-2.84 (2H, m), 2.71-2.63 (2H, m), 2.57-2.52 (1H, dd), 1.05-1.03 (3H, d), 0.83 (9H, s), 0.00 (3H, s), -0.19 (3H, s) ppm.
LRMS (electrospray) : m/z [M+H]⁺ 642, [M+Na]⁺ 664, [M-H]⁻ 640.

### Preparation 7: 2-(3-{(2R)-2-[(2R)-2-{[tert-Butyl(dimethyl)silyl]oxy}-2-(4-hydroxy-3-hydroxymethyl-phenyl)-ethylamino]-propyl}-phenyl)-N-(pyridin-2-ylmethyl)-acetamide

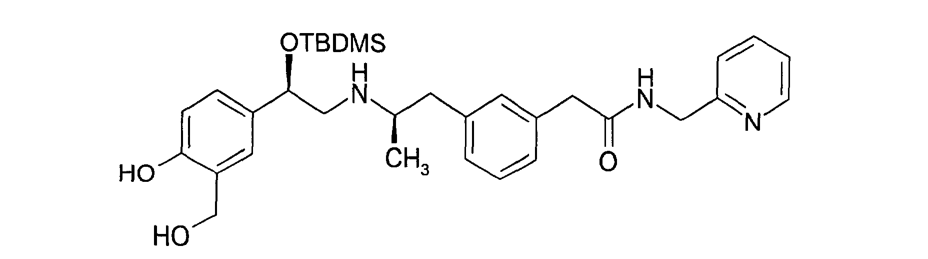

Prepared according to the procedure used for preparation 5 using the acid from preparation 22 and the appropriate amine to give the title compound as a colourless solid.
¹H NMR (400MHz, CD₃OD): δ = 8.44-8.42 (1H, d), 7.74-7.70 (1H, t), 7.27-7.23 (2H, m), 7.20-7.13 (3H, m), 7.06 (1H, s), 6.99-6.97 (2H, m), 6.68-6.66 (1H, d), 4.71-4.67 (1H, t), 4.61 (2H, d), 4.46 (2H, s), 3.55 (2H, s), 2.93-2.85 (2H, m), 2.71-2.63 (2H, m), 2.58-2.53 (1H, dd), 1.06-1.04 (3H, d), 0.84 (9H, s), 0.01 (3H, s), -0.18 (3H, s) ppm.
LRMS (electrospray) : m/z [M+H]⁺ 564, [M+Na]⁺ 586, [M-H]⁻ 562.

### Preparation 8: 4-{(2-(3-{(2R)-2-[(2R)-2-([tert-Butyl(dimethyl)silylloxy}-2-(4-hydroxy-3-hydroxymethyl-phenyl)-ethylamino]-propyl}-phenyl)-acetylamino)-methyl}-benzamide

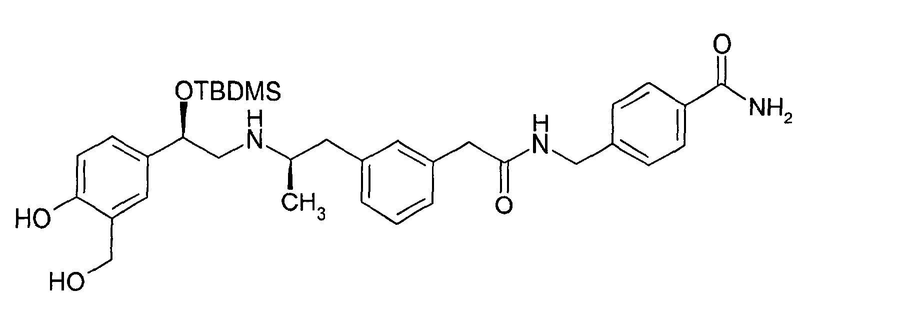

Prepared according to the procedure used for preparation 5 using the acid from preparation 22 and the appropriate amine to give the title compound as a colourless solid.
¹H NMR (400MHz, CD₃OD): δ = 7.79-7.77 (2H, d), 7.31-7.28 (2H, d), 7.20-7.11 (3H, m), 7.04 (1H, s), 6.99-6.97 (2H, m), 6.68-6.66 (1H, d), 4.71-4.69 (1H, t), 4.61-4.60 (2H, d), 4.40 (2H, s), 3.51 (2H, s), 2.92-2.83 (2H, m), 2.70-2.61 (2H, m), 2.58-2.53 (1H, m), 1.06-1.04 (3H, d), 0.83 (9H, s), 0.00 (3H, s), -0.18 (3H, s) ppm.
LRMS (electrospray) : m/z [M+H]⁺ 606, [M+Na]⁺ 628, [M-H]⁻ 604.

### Preparation 9: 2-(3-{(2R)-2-[(2R)-2-{[tert-Butyl(dimethyl)silyl]oxy}-2-(4-hydroxy-3-hydroxymethyl-phenyl}-ethylamino]-propyl)-phenyl)-N-(3,4-dimethoxybenzyl)-acetamide

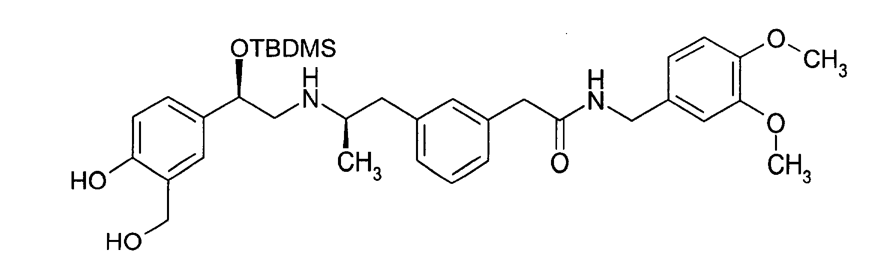

Prepared according to the procedure used for preparation 1 using the acid from preparation 22 and the appropriate amine to give the title compound as a colourless solid.
¹H NMR (400MHz, CD₃OD): δ = 7.20-7.13 (3H, m), 7.05 (1H, s), 7.00-6.98 (2H, d), 6.86-6.84 (1H, d), 6.79-6.77 (2H, m), 6.70-6.68 (1H, d), 4.72-4.68 (1H, m), 4.62-4.61 (2H, d), 4.29 (2H, s), 3.78 (3H, s), 3.71 (3H, s), 3.48 (2H, s), 2.94-2.83 (2H, m), 2.70-2.60 (2H, m), 2.57-2.51 (1H, m), 1.04-1.03 (3H, d), 0.83 (9H, s), 0.00 (3H, s), -0.19 (3H, s) ppm.
LRMS (electrospray) : m/z [M+H]⁺ 623, [M+Na]⁺ 645, [M-H]⁻ 621.

### Preparation 10: 2-(3-{(2R)-2-[(2R)-2-{[tert-Butyl(dimethyl)silyl]oxy}-2-(4-hydroxy-3-hydroxymethyl-phenyl)-ethylamino]-propyl}-phenyl)-N-(4-trifluoromethoxybenzyl)-acetamide

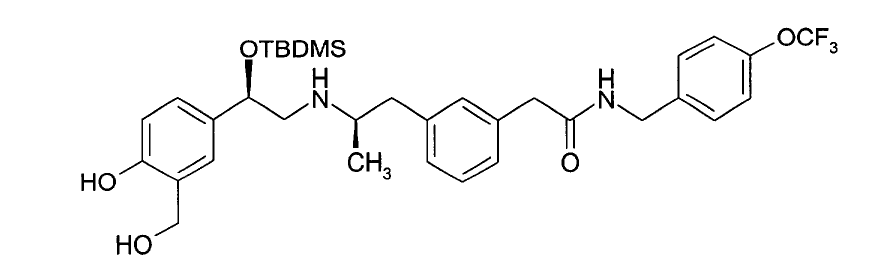

Prepared according to the procedure used for preparation 5 using the acid from preparation 22 and the appropriate amine to give the title compound as a colourless solid.
¹H NMR (400MHz, CD₃OD): δ = 7.32-7.30 (2H, d), 7.21-7.12 (5H, m), 7.05 (1H, s), 7.00-6.97 (2H, m), 6.69-6.67 (1H, d), 4.71-4.68 (1H, t), 4.60 (2H, d), 4.36 (2H, s), 3.49 (2H, s), 2.93-2.85 (2H, m), 2.70-2.60 (2H, m), 2.57-2.52 (1H, dd), 1.04-1.03 (3H, d), 0.83 (9H, s), 0.00 (3H, s), -0.20 (3H, s) ppm.
LRMS (electrospray) : m/z [M+H]⁺ 647, [M-H]- 645.

### Preparation 11: 2-(3-{(2R)-2-[(2R)-2-{[tert-Butyl(dimethyl)silyl]oxy}-2-(4-hydroxy-3-hydroxymethyl-phenyl)-ethylamino]-propyl}-phenyl)-N-(2-chloro, 6-fluorobenzyl)-acetamide

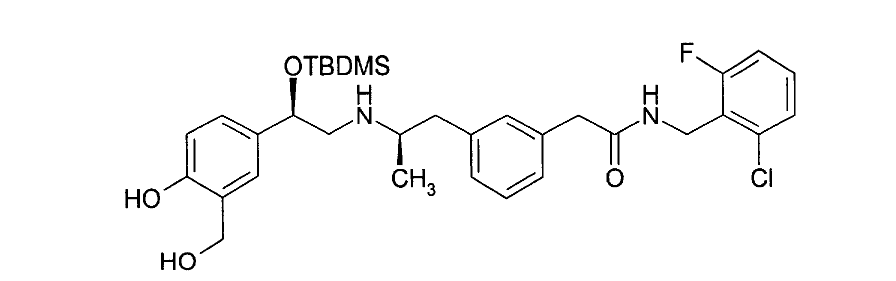

Prepared according to the procedure used for preparation 5 using the acid from preparation 22 and the appropriate amine to give the title compound as a colourless solid.
¹H NMR (400MHz, CD₃OD): δ = 7.33-7.14 (4H, m), 7.10-7.06 (2H, m), 7.02-6.95 (3H, m), 6.69-6.67 (1H, d), 4.72-4.68 (1H, t), 4.61-4.60 (2H, s), 4.52 (2H, s), 3.44 (2H, s), 2.93-2.85 (2H, m), 2.71-2.61 (2H, m), 2.54-2.49 (1H, dd), 1.04-1.02 (3H, d), 0.83 (9H, s), 0.00 (3H, s), -0.19 (3H, s) ppm.
LRMS (electrospray) : m/z [M+H]⁺ 615 / 617, [M+Na]⁺ 637 / 639, [M-H]⁻ 613 / 615.

### Preparation 12: 2-(3-{(2R)-2-[(2R)-2-{[tert-Butyl(dimethyl)silyl]oxy}-2-(4-hydroxy-3-hydroxymethyl-phenyl)-ethylamino]-propyl}-phenyl)-N-(3,4-dimethylbenzyl)-acetamide

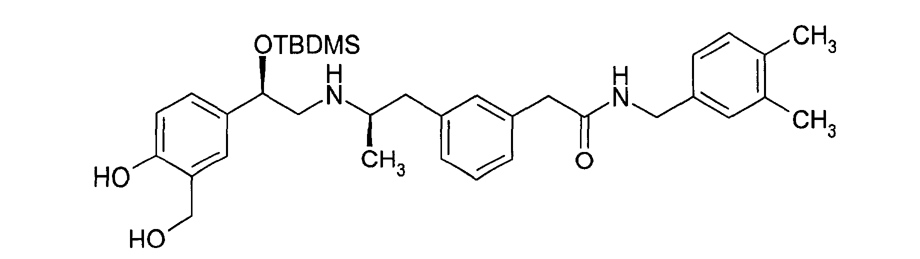

Prepared according to the procedure used for preparation 5 using the acid from preparation 22 and the appropriate amine to give the title compound as a colourless solid.
¹H NMR (400MHz, CD₃OD): δ = 7.19-7.11 (3H, m), 7.04-6.90 (6H, m), 6.68-6.66 (1H, d), 4.70-4.67 (1H, t), 4.61-4.60 (2H, d), 4.26 (2H, s), 3.47 (2H, s), 2.91-2.84 (2H, m), 2.69-2.60 (2H, m), 2.55-2.50 (1H, dd), 2.20 (3H, s), 2.18 (3H, s), 1.03-1.02 (3H, d), 0.82 (9H, s), -0.01 (3H, s), -0.20 (3H, s) ppm.
LRMS (electrospray) : m/z [M+H]⁺ 591, [M-H]⁻ 589.

### Preparation 13: 2-(3-{(2R)-2-[(2R)-2-{[tert-Butyl(dimethyl)silyl]oxy}-2-(4-hydroxy-3-hydroxymethyl-phenyl)-ethylamino]-propyl}-phenyl)-N-[2-fluoro-5-(trifluoromethyl)benzyl]-acetamide

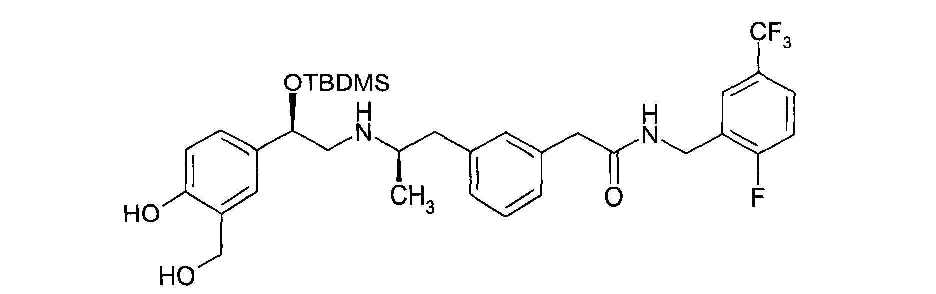

Prepared according to the procedure used for preparation 5 using the acid from preparation 22 and the appropriate amine to give the title compound as a colourless solid.
¹H NMR (400MHz, CD₃OD): δ = 7.61-7.58 (1H, m), 7.55-7.53 (1H, d), 7.28-7.12 (4H, m), 7.05 (1H, s), 7.02-6.97 (2H, m), 6.69-6.67 (1H, d), 4.72-4.69 (1H, t), 4.61 (2H, d), 4.45 (2H, s), 3.51 (2H, s), 2.93-2.84 (2H, m), 2.74-2.60 (2H, m), 2.57-2.51 (1H, dd), 1.04-1.03 (3H, d), 0.83 (9H, s), 0.00 (3H, s), -0.20 (3H, s) ppm.
LRMS (electrospray) : m/z [M+H]⁺ 649, [M-H]⁻ 647.

### Preparation 14: 2-(3-{(2R)-2-[(2R)-2-{[tert-Butyl(dimethyl)silyl]oxy}-2-(4-hydroxy-3-hydroxymethyl-phenyl)-ethylamino]-propyl}-phenyl)-N-(2,6-dichlorobenzyl)-acetamide

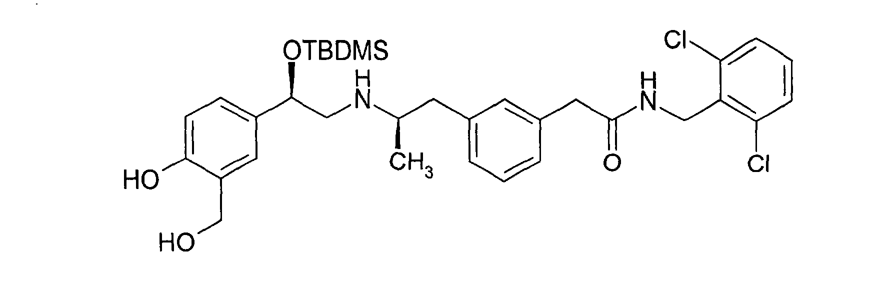

Prepared according to the procedure used for preparation 5 using the acid from preparation 22 and the appropriate amine to give the title compound as a colourless solid.
¹H NMR (400MHz, CD₃OD): δ = 7.35-7.33 (2H, m), 7.25-7.21 (1H, m), 7.17 (1H, s), 7.14-7.10 (1H, m), 7.07-7.05 (1H, m), 7.00 (1H, s), 6.97-6.91 (2H, m), 6.66-6.64 (1H, d), 4.68-4.65 (1H, t), 4.61 (2H, s), 4.58-4.57 (2H, d), 3.42 (2H, s), 2.89-2.82 (2H, m), 2.67-2.60 (2H, m), 2.51-2.47 (1H, dd), 1.00-0.99 (3H, d), 0.80 (9H, s), -0.04 (3H, s), -0.23 (3H, s) ppm.
LRMS (electrospray) : m/z [M+H]⁺ 631 / 633, [M+Na]⁺ 653 / 655, [M-H]⁻ 629 / 631.

### Preparation 15: 2-(3-{(2R)-2-[(2R)-2-{[tert-Butyl(dimethyl)silyl]oxy}-2-(4-hydroxy-3-hydroxymethyl-phenyl)-ethylamino]-propyl}-phenyl)-N-(2-phenylethyl)acetamide

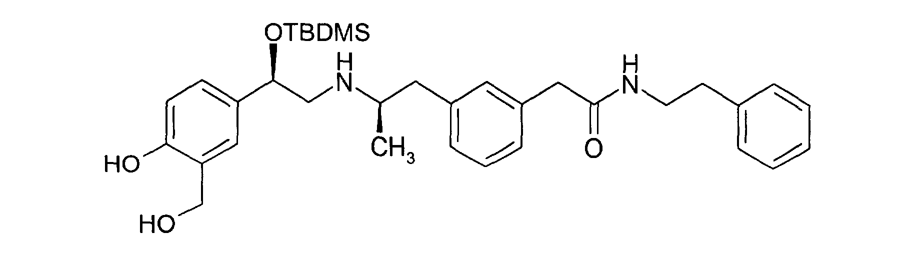

Prepared according to the procedure used for preparation 5 using the acid from preparation 22 and the appropriate amine to give the title compound as a brown oil.
¹H NMR (400MHz, CD₃OD): δ = 7.31-7.11 (7H, m), 7.07-7.05 (1H, d), 6.99-6.97 (3H, m), 6.68-6.66 (1H, d), 4.70-4.67 (1H, t), 4.61-4.60 (2H, d), 3.41-3.37 (2H, m), 3.34 (2H, s), 2.95-2.84 (2H, m), 2.81-2.73 (2H, m), 2.68-2.61 (2H, m), 2.56-2.51 (1H, dd), 1.05-1.03 (3H, d), 0.82 (9H, s), -0.01 (3H, s), -0.20 (3H, s) ppm.
LRMS (electrospray) : m/z [M+H]⁺ 577, [M+Na]⁺ 599, [M-H]⁻ 575.

### Preparation 16: N-Benzyl-2-(3-{(2R)-2-[(2R)-2-{[tert-Butyl(dimethyl)silyl] oxy}-2-(4-hydroxy-3-hydroxymethyl-phenyl)-ethylamino]-propyl}-phenyl)-acetamide

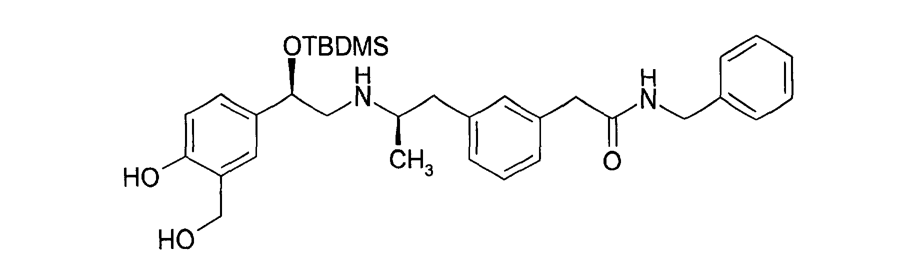

Prepared according to the procedure used for preparation 5 using the acid from preparation 22 and the appropriate amine to give the title compound as a colourless oil.
¹H NMR (400MHz, CD₃OD): δ = 7.35-7.12 (8H, m), 7.04 (1H, s), 6.99-6.97 (2H, d), 6.69-6.67 (1H, d), 4.70-4.67 (1H, t), 4.61-4.60 (2H, d), 4.35 (2H, s), 3.49 (2H, s), 2.90-2.83 (2H, m), 2.69-2.61 (2H, m), 2.55-2.50 (1H, dd), 1.04-1.02 (3H, d), 0.83 (9H, s), 0.00 (3H, s), -0.20 (3H, s) ppm.
LRMS (electrospray) : m/z [M+Na]⁺ 585, [M-H]⁻ 561.

### Preparation 17: 2-(3-{(2R)-2-[(2R)-2-{[tert-Butyl(dimethyl)silyl]oxy}-2-(4-hydroxy-3-hydroxymethyl-phenyl)-ethylamino]-propyl}-phenyl)-N-(3,5-dichlorobenzyl)-acetamide

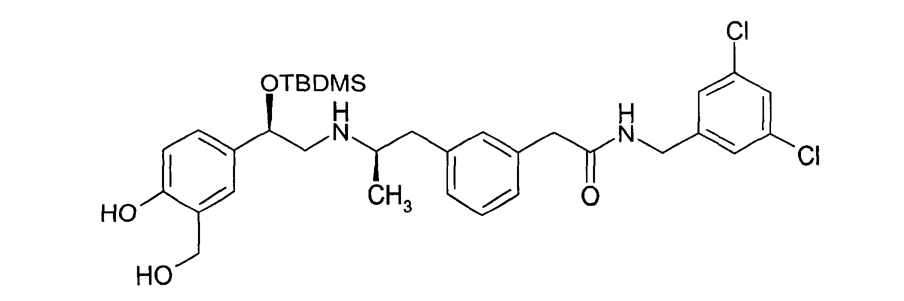

Prepared according to the procedure used for preparation 5 using the acid from preparation 22 and the appropriate amine to give the title compound as a colourless oil.
¹H NMR (400MHz, CD₃OD): δ = 7.33-7.28 (2H, m), 7.22-7.12 (4H, m), 7.05 (1H, s), 7.01-6.98 (2H, m), 6.69-6.67 (1H, d), 4.71-4.68 (1H, t), 4.62-4.61 (2H, d), 4.32 (2H, s), 3.51 (2H, s), 2.91-2.84 (2H, m), 2.71-2.61 (2H, m), 2.57-2.52 (1H, dd), 1.04-1.03 (3H, d), 0.83 (9H, s), 0.00 (3H, s), -0.19 (3H, s) ppm.
LRMS (electrospray) : m/z [M+H]⁺ 631 / 633, [M+Na]⁺ 653 / 655, [M-H]⁻ 629 / 631.

### Preparation 18: 2-(3-((2R)-2-[(2R)-2-{[tert-Butyl(dimethyl)silyl]oxy}-2-(4-hydroxy-3-hydroxymethyl-phenyl)-ethylamino]-propyl}-phenyl)-N-(4-chlorobenzyl)-acetamide

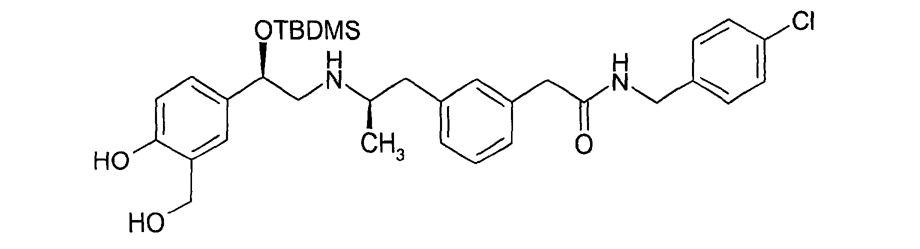

Prepared according to the procedure used for preparation 5 using the acid from preparation 22 and the appropriate amine to give the title compound as a colourless oil.
¹H NMR (400MHz, CD₃OD): δ = 7.31 (1H, s), 7.28-7.25 (1H, d), 7.21-7.16 (4H, m), 7.13-7.11 (1H, m), 7.03 (1H, s), 6.99-6.97 (2H, d), 6.69-6.67 (1H, d), 4.70-4.67 (1H, t), 4.62-4.62 (2H, d), 4.32 (2H, s), 3.48 (2H, s), 2.88-2.83 (2H, m), 2.68-2.60 (2H, m), 2.55-2.50 (1H, dd), 1.03-1.02 (2H, d), 0.83 (9H, s), -0.01 (3H, s), -0.20 (3H, s) ppm.
LRMS (electrospray) : m/z [M+H]⁺ 597 / 599, [M+Na]⁺ 619 / 621, [M-H]⁻ 595 / 597.

### Preparation 19: 4-{([2-(3-{(2R)-2-[(2R)-2-([tert-Butyl(dimethyl)silyl]oxy}-2-(4-hydroxy-3-hydroxymethyl-phenyl)-ethylamino]-propyl}-phenyl)-acetyl]-amino)-methyl}-benzoic acid

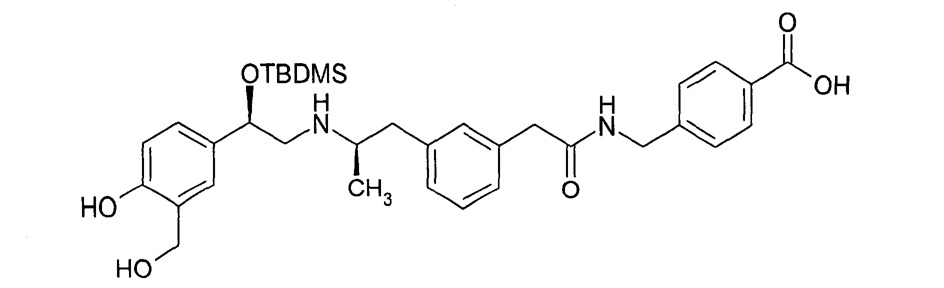

Prepared according to the procedure used for preparation 22, using the ester from preparation 20, to give the title compound as a colourless solid which was used without further purification.
¹H NMR (400MHz, CD₃OD): δ = 7.89-7.87 (2H, d), 7.34-7.20 (5H, m), 7.15-7.09 (3H, m), 6.80-6.78 (1H, d), 5.00-4.97 (1H, m), 4.67-4.66 (2H, d), 4.40 (2H, s), 3.55 (2H, s), 3.45-3.38 (1H, m), 3.28-3.23 (1H, dd), 3.15-3.11 (1H, dd), 3.03-2.98 (1H, dd), 2.72-2.66 (1H, dd), 1.17-1.16 (3H, d), 0.86 (9H, s), 0.07 (3H, s), -0.12 (3H, s) ppm.
LRMS (electrospray) : m/z [M+H]⁺ 607, [M+Na]⁺ 629, [M-H]⁻ 605.
CHN analysis : found C 62.04%, H 7.50% N 3.79%;
C₃₄H₄₆N₂O₆Si+0.5MeOH+2.6H₂O requires C 61.97%, H 7.87%, N 4.19%.

### Preparation 20: Methyl 4-{([2-(3-{(2R)-2-[(2R)-2-{[tert-butyl(dimethyl) silyl]oxy}-2-(4-hydroxy-3-hydroxymethyl-phenyl)-ethylamino]-propyl}-phenyl)-acetyl]-amino)-methyl}-benzoate

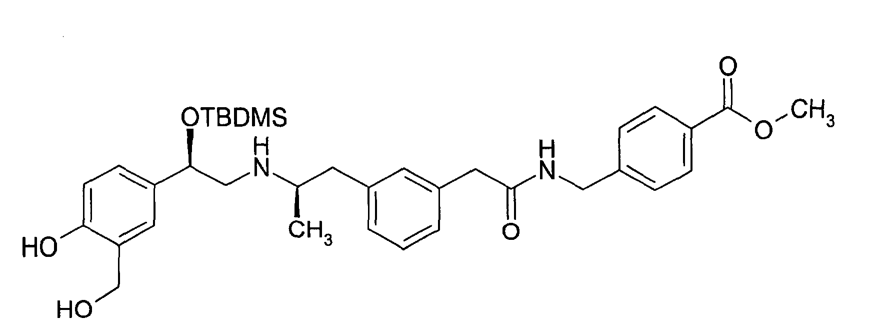

Prepared according to the procedure used for preparation 5 using the acid from preparation 22 and the appropriate amine to give the title compound as an orange oil.
¹H NMR (400MHz, CD₃OD): δ = 7.93-7.91 (2H, d), 7.33-7.31 (2H, d), 7.21-7.13 (3H, m), 7.05 (1H, s), 7.00-6.97 (2H, m), 6.69-6.67 (1H, d), 4.70-4.67 (1H, m), 4.62-4.61 (2H, d), 4.42 (2H, s), 3.87 (3H, s), 3.52 (2H, s), 2.91-2.84 (2H, m), 2.69-2.60 (2H, m), 2.56-2.51 (1H, dd), 1.04-1.02 (3H, d), 0.84 (9H, s), 0.01 (3H, s), -0.19 (3H, s) ppm.
LRMS (electrospray) : m/z [M+H]⁺ 621.

### Preparation 21: 3-(3-{(2R)-2-[(2R)-2-{[tert-Butyl(dimethyl)silyl]oxy}-2-(4-hydroxy-3-hydroxymethyl-phenyl}-ethylamino]-propyl)-phenyl)-N-(2,6-dichlorobenzyl)-propanamide

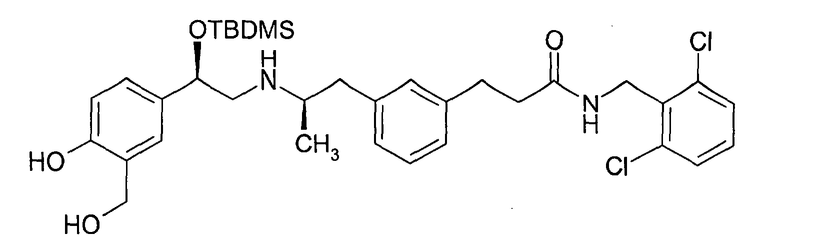

Prepared using the method for preparation (5) using the acid from preparation 31 and the appropriate amine to give the title compounds as a colourless solid. ¹H NMR (400MHz, CD₃OD): δ = 7.39-7.37 (2H, d), 7.28-7.24 (1H, m), 7.20-7.19 (1H, d), 7.14-7.10 (1H, t), 7.03-6.97 (2H, m), 6.94-6.90 (2H, m), 6.69-6.67 (1H, d), 4.73-4.69 (1H, t), 4.64-4.56 (4H, m), 2.95-2.84 (4H, m), 2.69-2.63 (2H, m), 2.56-2.50 (1H, m), 2.48-2.44 (2H, t), 1.06-1.04 (3H, d), 0.82 (9H, s), -0.01 (3H, s), -0.19 (3H, s) ppm.
LRMS (electrospray) : m/z [M+H]⁺ 645 / 647, [M+Na]⁺ 667 / 669, [M-H]- 643 / 645.

### Preparation 22: (3-{(2R)-2-[(2R)-2-{[tert-Butyl(dimethyl)silyl]oxy}-2-(4-hydroxy-3-hydroxymethyl-phenyl)-ethylamino]-propyl}-phenyl)-acetic acid

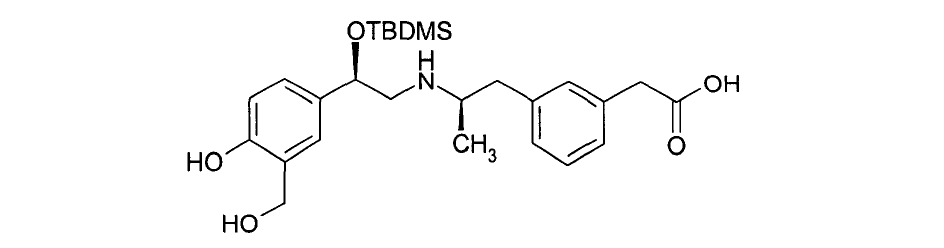

A solution of the ester from preparation 23 (7.04g, 14.43mmol) in tetrahydrofuran (40ml) was treated with lithium hydroxide (28.9ml of a 1M aqueous solution, 28.9mmol) and the reaction left to stir at room temperature for 16 hours. Hydrochloric acid (28.9ml of a 1M aqueous solution, 28.9mmol) was added and then the tetrahydrofuran was removed *in vacuo.* The remaining aqueous layer was decanted and the residue washed with further water (10ml). The residue was redisolved in methanol (30ml) and the solvent removed *in vacuo* to give the title compound as a colorless foam (5.95g) which was used without further purification.
¹H NMR (400MHz, CD₃OD): δ = 7.32 (1H, s), 7.25-7.18 (2H, m), 7.13 (1H, s), 7.12-7.10 (1H, d), 7.02-7.01 (1H, d), 6.79-6.77 (1H, d), 4.98-4.95 (1H, m), 4.65-4.64 (2H, d), 3.48 (2H, s), 3.48-3.43 (1H, m), 3.28-3.23 (1H, dd), 3.13-3.09 (1H, dd), 2.98-2.93 (1H, dd), 2.77-2.72 (1H, dd), 1.23-1.21 (3H, d), 0.86 (9H, s), 0.06 (3H, s), -0.13 (3H, s) ppm.
LRMS (electrospray) : m/z [M+H]⁺ 474, [M+Na]⁺ 496, [M-H]⁻ 472.
CHN analysis : found C 64.15%, H 8.25%, N 2.84%; C₂₆H₃₉NO₅Si+0.7H₂O requires C 64.22%, H 8.37%, N 2.88%.

### Preparation 23: Methyl (3-{(2R}-2-[{2R}-2-{[tert-butyl(dimethyl)silyl]oxy}-2-(4-hydroxy-3-hydroxymethyl-phenyl)-ethylamino]-propyl}-phenyl)-acetate

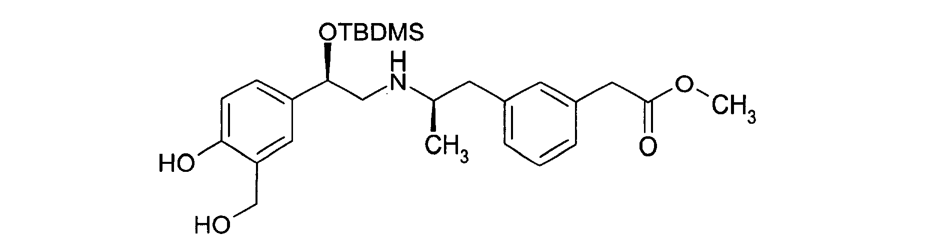

A suspension of preparation 24 (5.27g, 9.12mmol) and 10% palladium on carbon (1.00g) in ethanol (50ml) was stirred under an atmosphere of hydrogen (60psi) at room temperature for 16 hours. The catalyst was filtered off through arbocel and the filtrate concentrated *in vacuo.* The residue was purified by flash column chromatography on silica gel eluting with dichloromethane:methanol:880 ammonia (96:4:0.4 changing to 95:5:0.5, by volume) to give the title compound as a pale yellow oil (1.99g) which was used without further purification.
¹H NMR (400MHz, CD₃OD): δ = 7.21-7.17 (2H, m), 7.11-7.09 (1H, d), 7.03-6.98 (3H, m), 6.69-6.67 (1H, d), 4.71-4.68 (1H, t), 4.62-4.61 (2H, d), 3.67 (3H, s), 3.59 (2H, s), 2.96-2.86 (2H, m), 2.69-2.55 (3H, m), 1.07-1.05 (3H, d), 0.82 (9H, s), -0.01 (3H, s), -0.20 (3H, s) ppm.
LRMS (electrospray) : m/z [M+H]⁺ 488, [M+Na]⁺ 510, [M-H]⁻ 486

### Preparation 24: Methyl (3-{(2R)-2-[(2R)-2-{[tert-butyl(dimethyl)silyl]oxy}-2-(4-[benzyloxy]-3-hydroxymethyl-phenyl)-ethylamino]-propyl}-phenyl)-acetate

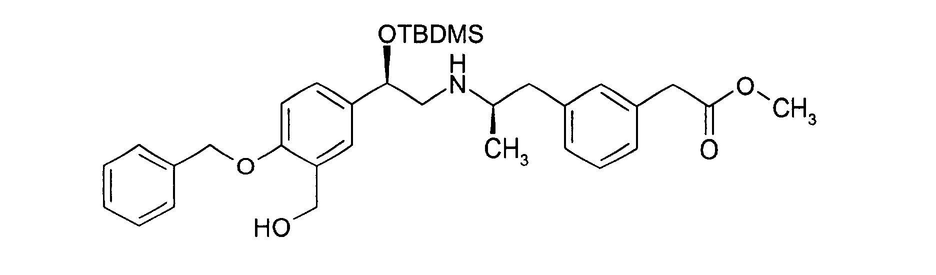

A solution of the bromide from preparation 25 (12.5g, 27.7mmol) and the amine from preparation 27 (11.5g, 55.4mmol) in dichloromethane (130ml) was heated to 90°C, allowing the dichloromethane to evaporate. The resulting melt was left at 90°C for a further 16 hours. The reaction mixture was cooled to room temperature and purified by flash column chromatography on silica gel eluting with dichloromethane:methanol:880 ammonia (98:2:0.2 changing to 97:3:0.3, by volume) to give the title compound (12.1g) as a colourless oil.
¹H NMR (400MHz, CD₃OD): δ = 7.47-7.45 (2H, m), 7.39-7.29 (4H, m), 7.19-7.15 (1H, t), 7.13-7.07 (2H, m), 7.03 (1H, s), 7.01-6.99 (1H, d), 6.93-6.91 (1H, d), 5.12 (2H, s), 4.76-4.73 (1H, t), 4.67-4.66 (2H, d), 3.66 (3H, s), 3.58 (2H, s), 2.95-2.80 (2H, m), 2.68-2.55 (3H, m), 1.06-1.05 (3H, d), 0.83 (9H, s), 0.00 (3H, s), -0.19 (3H, s) ppm.
LRMS (electrospray) : m/z [M+H]⁺ 578, [M+Na]⁺ 600.

### Preparation 25: [2-(Benzyloxy)-5-((1R)-2-bromo-1-{[tert-butyl(dimethyl) silyl]oxy}ethyl)phenyl]methanol

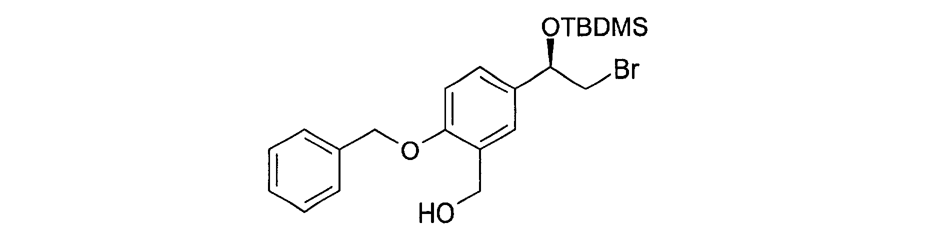

Borane dimethylsulfide complex (42.4ml of ∼10M solution, 424mmol) was added dropwise to a solution of preparation 26, (91.0g, 189mmol) in tetrahydrofuran (1600ml). The resulting mixture was then heated to reflux for 2 hours and then cooled to 0°C before quenching with methanol (270ml). The mixture was left to stir at room temperature for 16 hours and then the solvent removed *in vacuo.* The residue was partitioned between dichloromethane (500ml) and water (500ml). The aqueous phase was separated and extracted with dichloromethane (500ml) and the combined organic extracts washed with saturated aqueous sodium chloride (500ml), dried (magnesium sulfate) and the solvent removed *in vacuo.* The residue was purified by flash column chromatography on silica gel eluting with cyclohexane:ethyl acetate (100:0 changing to 80:20, by volume) to give the title compound (68.7g) as a colourless oil.
¹H NMR (400MHz, CDCl₃): δ = 7.42-7.36 (5H, m), 7.29-7.25 (3H, m), 6.94 (1H, d), 5.12 (2H, s), 4.84-4.81 (1H, m), 4.74 (2H, s), 3.48-3.40 (2H, m), 0.90 (9H, s), 0.11 (3H, s), -0.07 (3H, s) ppm.
LRMS (electrospray) : m/z [M+Na]⁺ 473 / 475.

### Preparation 26: Methyl 2-(benzyloxy)-5-((1R)-2-bromo-1-{[tert-butyl (dimethyl)silyl]oxy}ethyl)benzoate

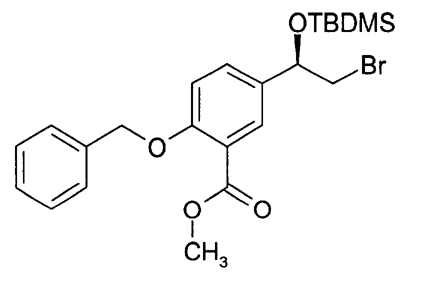

A solution of methyl 2-(benzyloxy)-5-[(1*R*)-2-bromo-1-hydroxyethyl]benzoate (71.05g, 195mmol), imidazole (18.52g, 272mmol), *tert*-butyldimethylsilyl chloride (32.23g, 214mmol) and 4-(*N,N*-dimethylamino)pyridine (0.44g, 3.6mmol) in *N,N*-dimethylformamide (270ml) was left to stir at room temperature under a nitrogen atmosphere for a period of 24 hours. The solvent was removed *in vacuo* and the residue partitioned between ethyl acetate (500ml) and water (500ml). The organic phase was separated and washed with 2N hydrochloric acid (2-fold 500ml), saturated aqueous sodium bicarbonate (2-fold 500ml) saturated sodium chloride (500ml), dried (magnesium sulfate) and the solvent removed *in vacuo* to give the title compound as a colourless oil (91.0g).
¹H NMR (400MHz, CDCl₃): δ = 7.81 (1H, bs), 7.51-7.30 (6H, m), 7.01 (1H, d), 5.19 (2H, s), 4.85-4.82 (1H, m), 3.91 (3H, s), 3.48-3.39 (2H, m), 0.90 (9H, s), 0.11 (3H, s), -0.08 (3H, s) ppm.
LRMS (electrospray) : m/z [M+Na]⁺ 501 / 503.

### Preparation 27: Methyl {3-[(2R)-2-aminopropyl]phenyl}acetate

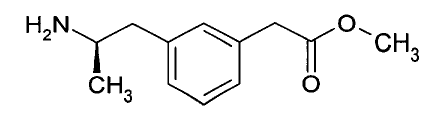

A solution of preparation 28 (7.69g, 22mmol) and ammonium formate (6.94g, 110mmol) was heated to 75°C in the presence of 20% palladium hydroxide-on-charcoal (Pd(OH)₂/C, 2.00g). After 90 minutes the reaction mixture was cooled to room temperature, filtered through arbocel and the filtrate concentrated *in vacuo.* The residue was partitioned between dichloromethane (100ml) and 880 ammonia (100ml) and the organic phase separated. The aqueous phase was extracted dichloromethane (100ml) and the combined organic extracts dried (magnesium sulfate) and reduced *in vacuo* to give the title compound as a colourless oil (4.78g).
¹H NMR (400MHz, CD₃OD): δ = 7.27-7.23 (1H, t), 7.13-7.09 (3H, m), 3.67 (3H, s), 3.63 (2H, s), 3.12-3.05 (1H, m), 2.67-2.57 (2H, m), 1.06 (3H, d) ppm.
LRMS (electrospray) : m/z [M+H]⁺ 208, [M+Na]⁺ 230.

### Preparation 28: Methyl [3-((2R)-2-{[(1R)-1-phenyl-ethyl]-amino}-propyl)-phenyl]-acetate hydrochloride

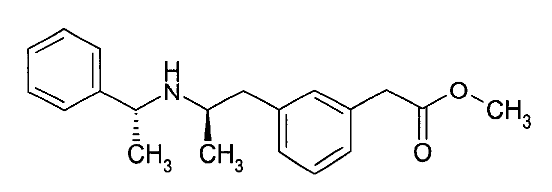

A solution of preparation 29 (8.5g, 41.2mmol), (*R*)-α-methyl benzylamine (4.8ml, 37.2mmol), sodium triacetoxyborohydride (11.6g, 56mmol) and acetic acid (2.2ml, 38mmol) in dichloromethane (400ml) was stirred at room temperature for 48 hours. The reaction mixture was quenched by addition of saturated aqueous sodium bicarbonate (200ml) and allowed to stir until effervescence ceased. The organic phase was separated and the aqueous phase extracted with dichloromethane (100ml). The combined organic extracts were dried (magnesium sulfate) and reduced *in vacuo.* Purification by flash column chromatography eluting with dichloromethane:methanol: ammonia (99:1:0.1 to 95:5:0.5 by volume) gave a 4:1 mixture of diastereomers (*R,R* major) as a pale yellow oil (8.71 g). Treatment with hydrogen chloride (40ml of a 1M solution in methanol, 40mmol) followed by three successive crystallisations (diisopropylether/ methanol) gave the title compound as a colourless crystalline solid (5.68g).
¹H NMR (400MHz, CD₃OD): δ = 7.52-7.48 (5H, m), 7.28-7.25 (1H, m), 7.18-7.16 (1H, m), 7.02-6.99 (2H, m), 4.59 (1H, q), 3.62 (2H, s), 3.30 (3H, s), 3.30-3.25 (1H, m), 3.26-3.15 (1H, m), 2.66-2.60 (1H, m), 1.68 (3H, d), 1.18, (3H, d) ppm.
LRMS (electrospray): m/z [M+H]⁺ 312, [M+Na]⁺ 334.

### Preparation 29: Methyl [3-(2-oxopropyl)pheny)]acetate

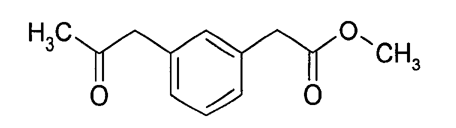

Tributyltin methoxide (28.3 ml, 98 mmol), preparation 30 (15.0g, 65.0mmol), isopropenyl acetate (10.8ml, 98.0mmol), palladium(II)acetate (750mg, 3.30mmol) and tri-*ortho*-tolylphosphine (2.0g, 6.5mmol) were stirred together in toluene (75ml) at 100°C under nitrogen for 5 hours. After cooling the reaction was diluted with ethyl acetate (150ml) and 4M aqueous potassium fluoride solution (90ml) and stirred for 15 minutes. The mixture was filtered through arbocel and the organic phase separated and reduced *in vacuo*. The residue was purified by flash column chromatography silica gel eluting with a solvent gradient of diethyl ether:pentane (0:100 to 25:75, by volume) changing to dichloromethane to give the title compound as a pale yellow oil (12.6g).
¹H NMR (400MHz, CDCl₃): δ = 7.30 (1H, t), 7.19 (1H, d), 7.13-7.10 (2H, m), 3.69 (5H, s), 3.61 (2H, s), 2.15 (3H, s) ppm.
LRMS (electrospray) : m/z [M+NH₄]⁺ 224, [M+Na]⁺ 229.

### Preparation 30: Methyl (3-bromophenyl)acetate

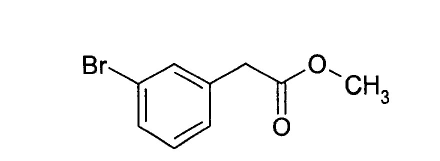

Acetyl chloride (0.7ml, 9.3mmol) was slowly added to a solution of (3-bromo-phenyl)-acetic acid (20.0g, 93mmol) in methanol (500ml) at 0°C under nitrogen and the reaction was allowed to warm gradually to room temperature over a period of 5 hours. The solvent was removed *in vacuo* and the residual oil was redissolved in dichloromethane, dried (sodium sulfate) and concentrated *in vacuo* to give the title compound as a colourless oil (20.6g).
¹H NMR (400MHz, CDCl₃): δ = 7.37-7.45 (2H, m), 7.24-7.17 (2H, m), 3.70 (3H, s), 3.59 (2H, s) ppm.
LRMS (electrospray) : m/z [M+Na]⁺ 253.

### Preparation 31: 3-(3-{(2R)-2-((2R)-2-{[tert-Butyl(dimethyl)silyl]oxy}-2-(4-hydroxy-3-hydroxymethyl-phenyl)-ethylamino]-propyl}-phenyl)-propanoic acid

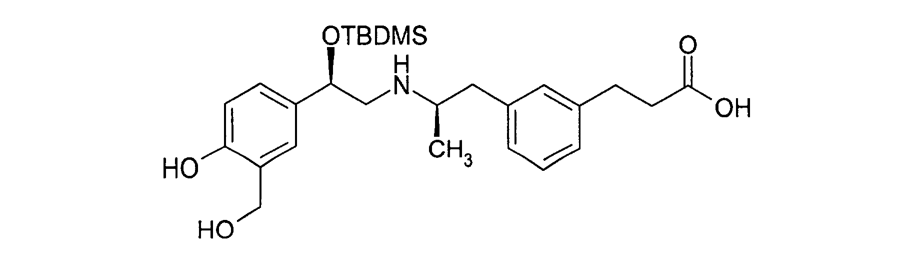

Prepared according to the procedure used for preparation 22, using the ester from preparation 32, to give the title compound as a brown gummy solid.

¹H NMR (400MHz, CD₃OD): δ = 7.27 (1H, s), 7.19-7.14 (1H, t), 7.09-7.02 (3H, m), 6.95-6.93 (1H, d), 6.75-6.73 (1H, d), 4.92-4.90 (1H, m), 4.61-4.60 (2H, d), 3.37-3.32 (1H, m), 3.20-3.15 (1H, m), 3.04-3.00 (1H, dd), 2.91-2.83 (3H, m), 2.68-2.62 (1H, m), 2.45-2.40 (2H, t), 1.15-1.14 (3H, d), 0.81 (9H, s), -0.01 (3H, s), -0.18 (3H, s) ppm.
LRMS (electrospray) : m/z [M+H]⁺ 488, [M+Na]⁺ 510, [M-H]⁻ 486.

### Preparation 32: Methyl 3-(3-{(2R)-2-[(2R)-2-{[tert-butyl(dimethyl)silyl]oxy}-2-(4-hydroxy-3-hydroxymethyl-phenyl)-ethylamino]-propyl}-phenyl)-propanoate

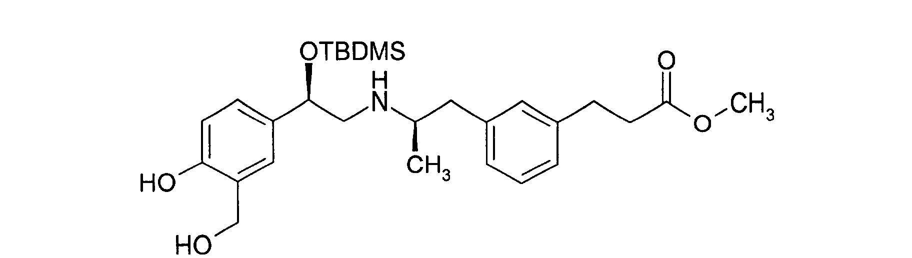

Prepared according to the procedure used for preparation 23, using the ester from preparation 33, to give the title compound as a brown oil.
¹H NMR (400MHz, CD₃OD): δ = 7.08-7.15 (2H, m), 6.99-6.88 (4H, m), 6.65-6.62 (1H, d), 4.67-4.64 (1H, m), 4.58-4.57 (2H, d), 3.59 (3H, s), 2.90-2.80 (4H, m), 2.61-2.49 (5H, m), 1.01-1.00 (3H, d), 0.78 (9H, s), -0.06 (3H, s), -0.24 (3H, s) ppm.
LRMS (electrospray) : m/z [M+H]⁺ 502, [M+Na]⁺ 524, [M-H]⁻ 500.

### Preparation 33: Methyl 3-(3-((2R)-2-[(2R)-2-([tert-Butyl(dimethyl)silyl] oxy}-2-(4-[benzyloxy]-3-hydroxymethyl-phenyl)-ethylamino]-propyl}-phenyl)-propanoate

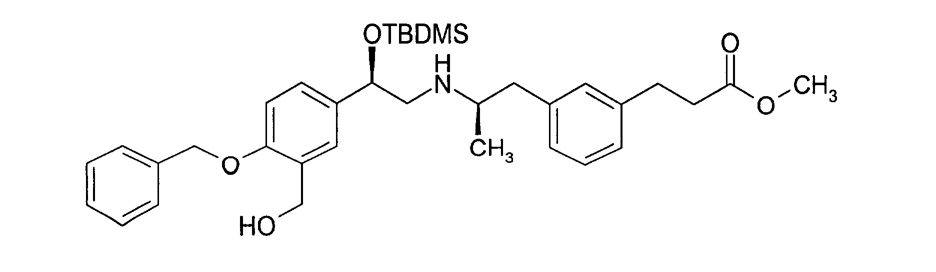

Prepared according to the procedure used for preparation 24 using the amine from preparation 34 and the bromide from preparation 25 to give the title compound as a brown oil.
¹H NMR (400MHz, CD₃OD): δ = 7.43-7.24 (6H, m), 7.11-7.04 (2H, m), 6.69-6.95 (1H, d), 6.91-6.86 (3H, m), 5.07 (2H, s), 4.71-4.66 (1H, m), 4.63-4.62 (2H, d), 3.58 (3H, s), 2.89-2.79 (4H, m), 2.63-2.47 (5H, m), 1.02-1.00 (3H, d), 0.78 (9H, s), -0.05 (3H, s), -0.23 (3H, s) ppm.
LRMS (electrospray) : m/z [M+H]⁺ 592, [M+Na]⁺ 614.

### Preparation 34: Methyl 3-[(2R)-2-aminopropyl)phenyl]propanoate

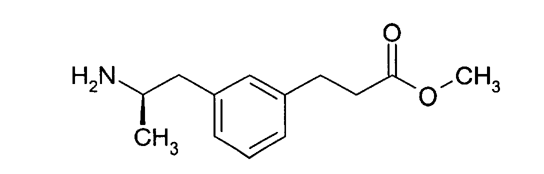

Prepared according to the procedure used for preparation 27, using the compound from preparation 35 to give the title compound as a brown oil.
¹H NMR (400MHz, CD₃OD): δ = 7.21-7.17 (1H, t), 7.03-7.01 (3H, m), 3.61 (3H, s), 3.11-3.03 (1H, m), 2.91-2.87 (2H, t), 2.64-2.54 (4H, m), 1.07-1.05 (3H, d) ppm.
LRMS (electrospray) : m/z [M+H]⁺ 222.

### Preparation 35: Methyl [3-((2R)-2-{[(1R)-1-phenyl-ethyl]-amino}-propyl)-phenyl]-propanoate hydrochloride

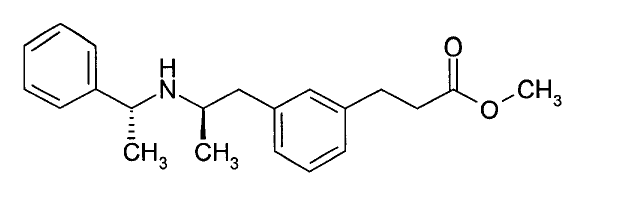

Prepared according to the procedure used for preparation 28, using the compound from preparation 36 to give the title compound as a colourless crystalline solid.
¹H NMR (400MHz, CD₃OD): δ = 7.54-7.47 (5H, m), 7.23-7.19 (1H, t), 7.12-7.10 (1H, d), 6.92-6.91 (2H, d), 4.64-4.59 (1H, q), 3.61 (3H, s), 3.34-3.29 (1H, m), 3.20-3.12 (1H, m), 2.89-2.85 (2H, t), 2.62-2.56 (3H, m), 1.71-1.69 (3H, d), 1.18-1.16 (3H, d) ppm.
LRMS (electrospray) : m/z [M+H]⁺ 326.

### Preparation 36: Methyl 3-[3-(2-oxopropyl)phenyl]propanoate

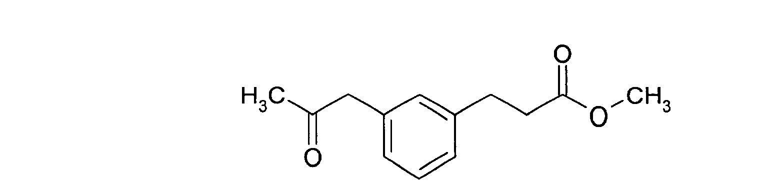

Prepared according to the procedure used for preparation 23 using the compound from preparation 35 to give the title compound as an orange oil.
¹H NMR (400MHz, CD₃OD): δ = 7.27-7.23 (1H, q), 7.11-7.09 (1H, d), 7.05-7.04 (2H, d), 3.66 (5H, s), 2.96-2.92 (2H, t), 2.64-2.60 (2H, t), 2.14 (3H, s) ppm.
LRMS (electrospray) : m/z [M+Na]⁺ 243, [M-H]⁻ 219.

### Preparation 37: Methyl (2E)-3-[3-(2-oxopropyl)phenyl]acrylate

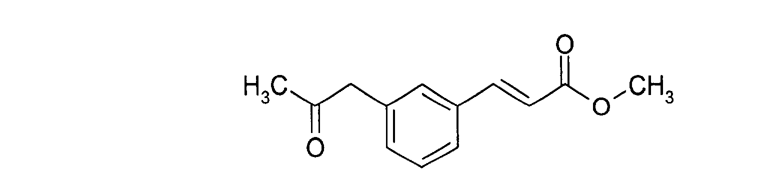

A solution of 3-bromophenylacetone (50.0g, 235.0mmol), methyl acrylate (40.4g, 469mmol), palladium(II)acetate (7.9g, 35.2mmol), tri-*ortho*-tolylphosphine (21.4g, 70.4mmol) and triethylamine (82.0ml, 58.7ml) in acetonitrile (900ml) was heated at reflux under a nitrogen atmosphere for a period of 16 hours. The reaction mixture was cooled to room temperature and the solvent removed *in vacuo.* Purification by flash column chromatography eluting with pentane : ethyl acetate (90:10 changing to 70:30 by volume) gave the title compound as an orange oil (54.3g).
¹H NMR (400MHz, CD₃OD): δ = 7.66-7.62 (1H, d), 7.41-7.39 (1H, d), 7.34-7.31 (2H, t), 7.20-7.18 (1H, d), 6.43-6.39 (1H, d), 3.77 (3H, s), 3.70 (2H, s), 2.15 (3H, s) ppm.
LRMS (electrospray) : m/z [M+Na]⁺ 241, [M-H]⁻ 217.

### Abbreviations

TBDMS = *tert*-butyl(dimethyl)silyl

### In vitro activity of the compounds of formula (1)

The ability of the compounds of the formula (1) to act as potent β2 agonists therefore mediating smooth muscle relaxation may be determined by the measure of the effect of beta-2 adrenergic receptor stimulation on electrical field stimulated-contraction of guinea pig trachea strips.

### Guinea-pig trachea

Male, Dunkin-Hartley guinea pigs (475-525g) are killed by CO₂ asphyxiation and exsanguination from the femoral artery and the trachea is isolated. Four preparations are obtained from each animal, starting the dissection immediately below the larynx and taking 2.5 cm length of trachea. The piece of trachea is opened by cutting the cartilage opposite the trachealis muscle, then transverse sections, 3-4 cartilage rings wide, are cut. The resulting strip preparations are suspended in 5 ml organ baths using cotton threads tied through the upper and lower cartilage bands. The strips are equilibrated, un-tensioned, for 20 minutes in a modified Krebs Ringer buffer (Sigma K0507) containing 3 µM Indomethacin (Sigma 17378), 10 µM Guanethidine (Sigma G8520) and 10 µM Atenolol (Sigma A7655), heated at 37°C and gassed with 95% O₂/5% CO₂, before applying an initial tension of 1 g. The preparations are allowed to equilibrate for a further 30-45 minutes, during which time they are re-tensioned (to 1 g) twice at 15-minute intervals. Changes in tension are recorded and monitored via standard isometric transducers coupled to a data-collection system (custom-designed at Pfizer). Following the tensioning equilibration, the tissues are subjected to electrical field stimulation (EFS) using the following parameters : 10 s trains every 2 minutes, 0.1 ms pulse width, 10 Hz and just-maximal voltage (25 Volts) continuously throughout the length of the experiment. EFS of post-ganglionic cholinergic nerves in the trachea results in monophasic contractions of the smooth muscle and twitch height is recorded. The organ baths are constantly perfused with the above-described Krebs Ringer buffer by means of a peristaltic pump system (pump flow rate 7.5 ml / minute) throughout the experiment, with the exception of when a beta-2 agonist according to the present invention is added, the pump is then stopped for the time of the cumulative dosing to the bath and started again after maximal response is reached for the wash-out period.

### Experimental protocol for assessment of potency and efficacy

Following equilibration to EFS, the peristaltic pump is stopped and the preparations 'primed' with a single dose of 300 nM isoprenaline (Sigma 15627) to establish a maximal response in terms of inhibition of the contractile EFS response. The isoprenaline is then washed out over a period of 40 minutes. Following the priming and wash-out recovery, a standard curve to isoprenaline is carried out on all tissues (Isoprenaline Curve 1) by means of cumulative, bolus addition to the bath using half log increments in concentration. The concentration range used is 1^{e-9} to 1^{e}/3^{e-6} M. At the end of the isoprenaline curve the preparations are washed again for 40 minutes before commencing a second curve, either to isoprenaline (as internal control) or a beta-2 agonist according to the present invention. Beta-2 agonist responses are expressed as percentage inhibition of the EFS response. Data for beta-2 agonist are normalised by expressing inhibition as a percentage of the maximal inhibition induced by isoprenaline in Curve 1. The EC₅₀ value for beta-2 agonist according to the present invention refers to the concentration of compound required to produce half maximal effect. Data for beta-2 agonists according to the present invention are then expressed as relative potency to isoprenaline defined by the ratio (EC₅₀ beta-2 agonist)/(EC50 Isoprenaline).

### Confirmation of beta-2 mediated functional activity

Beta-2 agonist activity of test compounds is confirmed using the protocol above, however, prior to constructing the curve to beta-2 agonist according to the present invention, the preparations are pre-incubated (for a minimum of 45 minutes) with 300 nM ICI 118551 (a selective β₂ antagonist) which results in the case of a beta-2 mediated effect in a rightward-shift of the test compound dose response curve.

It has thus been found that the compounds of formula (1) according to the present invention that have been tested show a relative potency to Isoprenaline which is comprised between 0.002 and 2.0.

According to another alternative, the agonist potency for the β2 receptor of the compounds of the formula (1) may also be determined by the measure of the concentration of compound according to the present invention required to produce half maximal effect (EC₅₀) for the β2 receptor.

### Compound Preparation

10 mM/100% DMSO (dimethylsulfoxide) stock of compound is diluted to required top dose in 4 % DMSO. This top dose is used to construct a 10-point semi-log dilution curve, all in 4 % DMSO. Isoprenaline (Sigma, I-5627) was used as a standard in every experiment and for control wells on each plate. Data was expressed as % Isoprenaline response.

### Cell Culture

CHO (Chinese Hamster Ovary) cells recombinantly expressing the human β2 adrenergic receptor (from Kobilka et al., PNAS 84: 46-50, 1987 and Bouvier et al., Mol Pharmacol 33: 133-139 1988 CHOhβ2) were grown in Dulbeccos MEM/NUT MIX F12 (Gibco, 21331-020) supplemented with 10 % foetal bovine serum (Sigma, F4135, Lot 90K8404 Exp 09/04), 2 mM glutamine (Sigma, G7513), 500 µg/ml geneticin (Sigma, G7034) and 10 µg/ml puromycin (Sigma, P8833). Cells were seeded to give about 90 % confluency for testing.

### Assay Method

25 µl / well each dose of compound was transferred into a CAMP- Flashplate® (NEN, SMP004B), with 1% DMSO as basal controls and 100 nM Isoprenaline as max controls. This was diluted 1:2 by the addition of 25 µl / well PBS. Cells were trypsinised (0.25% Sigma, T4049), washed with PBS (Gibco, 14040-174) and resuspended in stimulation buffer (NEN, SMP004B) to give 1x10⁶ cells / ml CHOhB2. Compounds were incubated with 50 µl / well cells for 1 hour. Cells were then lysed by the addition of 100 µl / well detection buffer (NEN, SMP004B) containing 0.18 µCi / ml ¹²⁵I-cAMP (NEN, NEX-130) and plates were incubated at room temperature for a further 2 hours. The amount of ¹²⁵I-cAMP bound to the Flashplate® was quantified using a Topcount NXT (Packard), normal counting efficiency for 1 minute. Dose-response data was expressed as % Isoprenaline activity and fitted using a four parameter sigmoid fit.

It has thus been found that the compounds of formula (1) according to the present invention that are illustrated in examples 1 to 20 above show a β2 cAMP EC₅₀ between 0.006 nM and 0.210 nM.

The results below illustrate the activity of the compounds of formula (1):

| Example Number | Cell based cAMP β2 activity (nM) |
|---|---|
| 1 | 0.017 |
| 3 | 0.007 |
| 4 | 0.012 |
| 5 | 0.024 |
| 6 | 0.007 |
| 7 | 0.028 |
| 8 | 0.010 |
| 10 | 0.114 |
| 12 | 0.009 |
| 13 | 0.013 |
| 15 | 0.056 |

## Claims

1. A compound of formula (1): 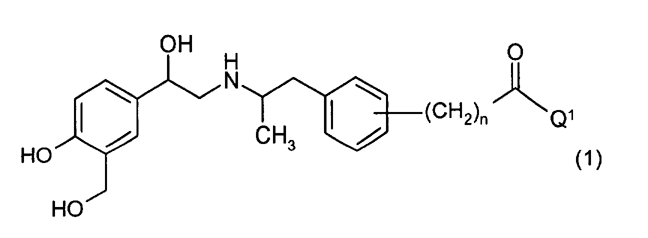 wherein the (CH₂)ₙ-C(=O)Q¹ group is in position meta or para, n is 1 or 2 and Q¹ is a group selected from: 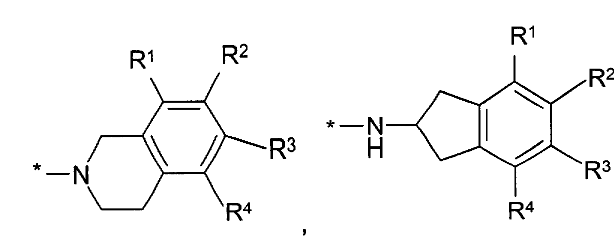 wherein at least 2 of R¹ to R⁴ are equal to H, and,
a group *-NH-Q²-A, wherein Q² is C₁-C₄ alkylene and A is pyridyl or a group of formula 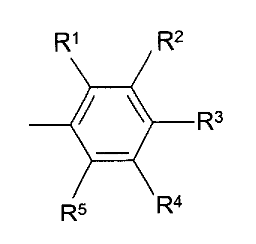 wherein at least 2 of R¹ to R⁵ are equal to H,
wherein R¹, R², R³, R⁴ and R⁵ are the same or different and are selected from H, C₁-C₄ alkyl, OR⁶, SR⁶, halo, CF₃, OCF₃, COOR⁶, SO₂NR⁶R⁷, CONR⁶R⁷, NR⁶R⁷, NHCOR⁶,
wherein R⁶ and R⁷ are the same or different and are selected from H or C₁-C₄ alkyl and the * represent the attachment point to the carbonyl group.
or, if appropriate, their pharmaceutically acceptable salts and/or isomers, tautomers, solvates or isotopic variations thereof.

2. A compound according to claim 1 wherein n is 1 or 2 and Q¹ is *-NH-Q²-A wherein Q² is C₁-C₄ alkylene and A is a group of formula 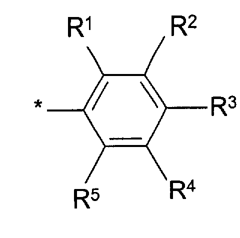 wherein R¹, R², R³, R⁴ and R⁵ are as defined in claim 1.

3. A compound according to claim 2 wherein R¹, R², R³, R⁴ and R⁵ are the same or different and are selected from H, C₁-C₄ alkyl, OR⁶, CI, F, CF₃, OCF₃, COOR⁶, SO₂NR⁶R⁷, provided at least 2 of R¹ to R⁵ are equal to H,
wherein R⁶ and R⁷ are the same or different and are selected from H or C₁-C₄ alkyl.

4. A compound according to claim 3 wherein R¹, R², R³, R⁴ and R⁵ are the same or different and are selected from H, CH₃, OH, OCH₃, OCH₂CH₃, CI, F, CF₃, OCF₃, COOH, SO₂NH₂, provided at least 2 of R¹ to R⁵ are equal to H.

5. A compound according to claim 4 wherein R¹, R², R³, R⁴ and R⁵ are the same or different and are selected from H, CH₃, OH, OCH₃, OCH₂CH₃, CI, F, CF₃, OCF₃, COOH, SO₂NH₂, provided at least 3 of R¹ to R⁵ are equal to H.

6. A compound according to claim 1 wherein Q¹ is a group *-NH-Q²-A, wherein Q² is C₁-C₄ alkylene and A is pyridin-2-yl.

7. A compound according to any one of claims 1 to 6 wherein Q² is selected from -CH₂-, -(CH₂)₂-, -(CH₂)₃- and -CH(CH₃)-.

8. A compound according to any one of claims 1 to 6 wherein Q² is -CH₂-.

9. A compound according to claim 1 wherein n is 1 or 2 and Q¹ is 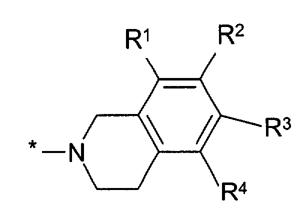 wherein R¹, R², R³ and R⁴ are the same or different and are selected from H, C₁-C₄ alkyl, OR⁶, SR⁶, halo, CF₃, OCF₃, COOR⁶, SO₂NR⁶R⁷, CONR⁶R⁷, NR⁶R⁷, NHCOR⁶, provided at least 2 of R¹ to R⁴ are equal to H;
wherein R⁶ and R⁷ are the same or different and are selected from H or C₁-C₄ alkyl.

10. A compound according to claim 9 wherein R¹, R², R³ and R⁴ are the same or different and are selected from H and OR⁷, provided at least 2 of R¹ to R⁴ are equal to H.

11. A compound according to claim 1 wherein Q¹ is

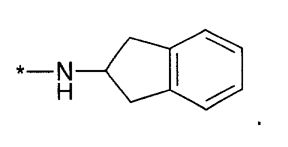

12. A compound according to any one of claims 1 to 11 wherein n is 1.

13. The (*R,R*)-stereoisomer of a compound according to any one of claims 1 to 12.

14. A compound according to any one of claims 1 to 13 wherein the (CH₂)ₙ-C(=O)Q¹ group is in position meta.

15. A compound according to claim 1 selected from the group consisting of
*N*-(2,6-Dimethoxybenzyl)-2-(3-{(2*R*)-2-[(2*R*)-2-hydroxy-2-(4-hydroxy-3-hydroxymethyl-phenyl)-ethylamino]-propyl}-phenyl)-acetamide,
*N*-(2-Ethoxybenzyl)-2-(3-{(2*R*)-2-[(2*R*)-2-hydroxy-2-(4-hydroxy-3-hydroxymethyl-phenyl)-ethylamino]-propyl}-phenyl)-acetamide,
*N*-(2-Hydroxybenzyl)-2-(3-{(2*R*)-2-[(2*R*)-2-hydroxy-2-(4-hydroxy-3-hydroxymethyl-phenyl)-ethylamino]-propyl}-phenyl)-acetamide,
2-(3-{(2*R*)-2-[(2*R*)-2-Hydroxy-2-(4-hydroxy-3-hydroxymethyl-phenyl)-ethylamino]-propyl}-phenyl)-*N*-indan-2-yl-acetamide,
*N*-(3,4-Dichlorobenzyl)-2-(3-{(2*R*)-2-[(2*R*)-2-hydroxy-2-(4-hydroxy-3-hydroxymethyl-phenyl)-ethylamino]-propyl}-phenyl)-acetamide,
*N*-[4-(Aminosulfonyl)benzyl]-2-(3-{(2*R*)-2-[(2*R*)-2-hydroxy-2-(4-hydroxy-3-hydroxymethyl-phenyl)-ethylamino]-propyl}-phenyl)-acetamide,
2-(3-{(2*R*)-2-[(2*R*)-2-Hydroxy-2-(4-hydroxy-3-hydroxymethyl-phenyl)-ethylamino]-propyl}-phenyl)- *N*-(pyridin-2-ylmethyl)-acetamide,
4-{(2-(3-{(2*R*)-2-[(2*R*)-2-Hydroxy-2-(4-hydroxy-3-hydroxymethyl-phenyl)-ethylamino]-propyl}-phenyl)-acetylamino)-methyl}-benzamide,
*N*-(3,4-Dimethoxybenzyl)-2-(3-{(2*R*)-2-[(2*R*)-2-hydroxy-2-(4-hydroxy-3-hydroxymethyl-phenyl)-ethylamino]-propyl}-phenyl)-acetamide,
2-(3-{(2*R*)-2-[(2*R*)-2-Hydroxy-2-(4-hydroxy-3-hydroxymethyl-phenyl)-ethylamino]-propyl}-phenyl)-*N*-(4-trifluoromethoxybenzyl)-acetamide,
*N*-(2-Chloro, 6-fluorobenzyl)-2-(3-{(2*R*)-2-[(2*R*)-2-hydroxy-2-(4-hydroxy-3-hydroxymethyl-phenyl)-ethylamino]-propyl}-phenyl)-acetamide,
*N*-(3,4-Dimethylbenzyl)-2-(3-{(2*R*)-2-[(2*R*)-2-hydroxy-2-(4-hydroxy-3-hydroxymethyl-phenyl)-ethylamino]-propyl}-phenyl)-acetamide,
*N*-[2-Fluoro-5-(trifluoromethyl)benzyl]-2-(3-{(2*R*)-2-[(2*R*)-2-hydroxy-2-(4-hydroxy-3-hydroxymethyl-phenyl)-ethylamino]-propyl}-phenyl)-acetamide,
*N*-(2,6-Dichlorobenzyl)-2-(3-{(2*R*)-2-[(2*R*)-2-hydroxy-2-(4-hydroxy-3-hydroxymethyl-phenyl)-ethylamino]-propyl)-phenyl)-acetamide,
2-(3-{(2*R*)-2-[(2*R*)-2-Hydroxy-2-(4-hydroxy-3-hydroxymethyl-phenyl)-ethylamino]-propyl}-phenyl)-*N*-(2-phenylethyl)acetamide,
*N*-Benzyl-2-(3-{(2*R*)-2-[(2*R*)-2-hydroxy-2-(4-hydroxy-3-hydroxymethyl-phenyl)-ethylamino]-propyl}-phenyl)-acetamide,
*N*-(3,5-Dichlorobenzyl)-2-(3-{(2*R*)-2-[(2*R*)-2-hydroxy-2-(4-hydroxy-3-hydroxymethyl-phenyl)-ethylamino]-propyl}-phenyl)-acetamide,
*N*-(4-Chlorobenzyl)-2-(3-{(2*R*)-2-[(2*R*)-2-hydroxy-2-(4-hydroxy-3-hydroxymethyl-phenyl)-ethylamino]-propyl}-phenyl)-acetamide,
4-{([2-(3-{(2*R*)-2-[(2*R*)-2-Hydroxy-2-(4-hydroxy-3-hydroxymethyl-phenyl)-ethylamino]-propyl}-phenyl)-acetyl]-amino)-methyl}-benzoic acid,
*N*-(2,6-Dichlorobenzyl)-3-(3-{(2*R*)-2-[(2*R*)-2-hydroxy-2-(4-hydroxy-3-hydroxymethyl-phenyl)-ethylamino]-propyl}-phenyl)-propanamide,
1-(3,4-Dihydro-1H-isoquinolin-2-yl)-2-(3-{(2*R*)-2-[(2*R*)-2-hydroxy-2-(4-hydroxy-3-hydroxymethyl-phenyl)-ethylamino]-propyl}-phenyl)-propan-1-one,
1-(3,4-Dihydro-1H-isoquinolin-2-yl)-2-(3-{(2*R*)-2-[(2*R*)-2-hydroxy-2-(4-hydroxy-3-hydroxymethyl-phenyl)-ethylamino]-propyl}-phenyl)-ethanone,
1-(7-Ethoxy-6-methoxy-3,4-dihydro-1H-isoquinolin-2-yl)-2-(3-{(2*R*)-2-[(2*R*)-2-hydroxy-2-(4-hydroxy-3-hydroxymethyl-phenyl)-ethylamino]-propyl}-phenyl)-ethanone,
3-(3-{(2*R*)-2-[(2*R*)-2-Hydroxy-2-(4-hydroxy-3-hydroxymethyl-phenyl)-ethylamino]-propyl}-phenyl)-N-indan-2-yl-propanamide,
N-(3,4-Dimethylbenzyl)-3-(3-{(2*R*)-2-[(2*R*)-2-hydroxy-2-(4-hydroxy-3-hydroxymethyl-phenyl)-ethylamino]-propyl}-phenyl)-propanamide,
N-(2,6-Dimethoxybenzyl)-3-(3-{(2*R*)-2-[(2*R*)-2-hydroxy-2-(4-hydroxy-3-hydroxymethyl-phenyl)-ethylamino]-propyl}-phenyl)-propanamide,
*N*-[(1*R*)-1-Phenylethyl]-2-(3-{(2*R*)-2-[(2*R*)-2-hydroxy-2-(4-hydroxy-3-hydroxymethyl-phenyl)-ethylamino]-propyl}-phenyl)-acetamide,
N-[2-(3,4-Dimethoxyphenyl)ethyl]-2-(3-{(2*R*)-2-[(2*R*)-2-hydroxy-2-(4-hydroxy-3-hydroxymethyl-phenyl)-ethylamino]-propyl}-phenyl)-acetamide,
N-[2-(4-Chlorophenyl)ethyl]-2-(3-{(2*R*)-2-[(2*R*)-2-hydroxy-2-(4-hydroxy-3-hydroxymethyl-phenyl)-ethylamino]-propyl}-phenyl)-acetamide,
N-[2-(4-Ethylphenyl)ethyl]-2-(3-{(2*R*)-2-[(2*R*)-2-hydroxy-2-(4-hydroxy-3-hydroxymethyl-phenyl)-ethylamino]-propyl}-phenyl)-acetamide,
N-(1,1-Dimethyl-2-phenylethyl)-2-(3-{(2*R*)-2-[(2*R*)-2-hydroxy-2-(4-hydroxy-3-hydroxymethyl-phenyl)-ethylamino]-propyl}-phenyl)-acetamide,
2-(3-{(2*R*)-2-[(2*R*)-2-Hydroxy-2-(4-hydroxy-3-hydroxymethyl-phenyl)-ethylamino]-propyl}-phenyl)-N-[3-(4-fluorophenyl)propyl]-acetamide,
N-(3-Chlorobenzyl)-2-(4-{(2*R*)-2-[(2*R*)-2-hydroxy-2-(4-hydroxy-3-hydroxymethyl-phenyl)-ethylamino]-propyl}-phenyl)-acetamide,
2-(4-{(2*R*)-2-[(2*R*)-2-Hydroxy-2-(4-hydroxy-3-hydroxymethyl-phenyl)-ethylamino]-propyl}-phenyl)-N-indan-2-yl-acetamide,
N-(2-Hydroxybenzyl)-2-(4-{(2*R*)-2-[(2*R*)-2-hydroxy-2-(4-hydroxy-3-hydroxymethyl-phenyl)-ethylamino]-propyl)-phenyl)-acetamide,
2-(4-{(2*R*)-2-[(2*R*)-2-hydroxy-2-(4-hydroxy-3-hydroxymethyl-phenyl)-ethylamino]-propyl}-phenyl)-N-(2-methoxybenzyl)-acetamide,
N-Benzyl-2-(4-{(2*R*)-2-[(2*R*)-2-hydroxy-2-(4-hydroxy-3-hydroxymethyl-phenyl)-ethylamino]-propyl}-phenyl)-acetamide,
N-(4-Chlorobenzyl)-2-(4-{(2*R*)-2-[(2*R*)-2-hydroxy-2-(4-hydroxy-3-hydroxymethyl-phenyl)-ethylamino]-propyl}-phenyl)-acetamide,
2-(4-{(2*R*)-2-[(2*R*)-2-hydroxy-2-(4-hydroxy-3-hydroxymethyl-phenyl)-ethylamino]-propyl}-phenyl)-N-(3-methoxybenzyl)-acetamide,
2-(4-{(2*R*)-2-[(2*R*)-2-Hydroxy-2-(4-hydroxy-3-hydroxymethyl-phenyl)-ethylamino]-propyl}-phenyl)-N-(pyridin-2-ylmethyl)-acetamide,
2-(4-{(2*R*)-2-[(2*R*)-2-hydroxy-2-(4-hydroxy-3-hydroxymethyl-phenyl)-ethylamino]-propyl}-phenyl)-N-(4-methoxybenzyl)-acetamide,
2-(4-{(2*R*)-2-[(2*R*)-2-Hydroxy-2-(4-hydroxy-3-hydroxymethyl-phenyl)-ethylamino]-propyl}-phenyl)-N-(3-phenylpropyl)-acetamide,
N-(2,6-Dimethoxybenzyl)-2-(4-{(2*R*)-2-[(2*R*)-2-hydroxy-2-(4-hydroxy-3-hydroxymethyl-phenyl)-ethylamino]-propyl}-phenyl)-acetamide, and,
1-(3,4-Dihydro-1H-isoquinolin-2-yl)-2-(4-{(2*R*)-2-[(2*R*)-2-hydroxy-2-(4-hydroxy-3-hydroxymethyl-phenyl)-ethylamino]-propyl}-phenyl)-ethanone.

16. A process for the preparation of a compound of formula (1) as described in claim 1 or a pharmaceutically acceptable salt or derived form thereof comprising the step of coupling an acid of formula (2): 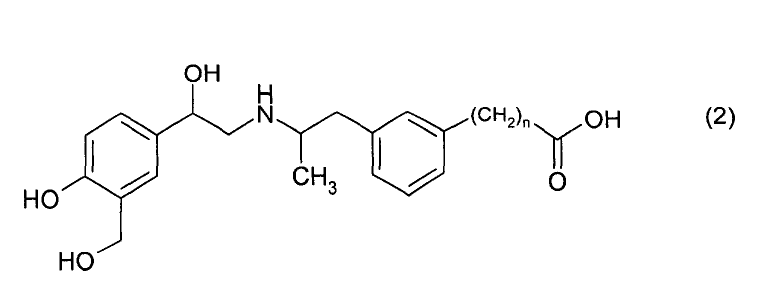 with an amine of formula NH₂-Q²-A (3), 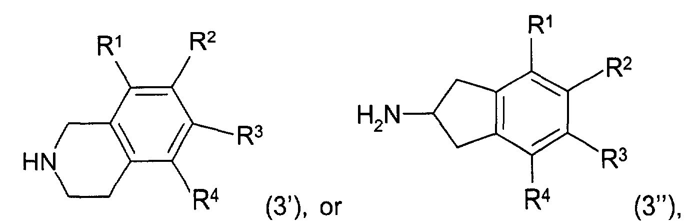 wherein n, Q¹, Q², A, R¹, R², R³ and R⁴ are as defined in claim 1.

17. A pharmaceutical composition including a compound of formula (1) as described in claim 1 or a pharmaceutically acceptable salt or derived form thereof, together with customary pharmaceutically innocuous excipients and/or additives.

18. A compound of formula (1) as described in claim 1 or a pharmaceutically acceptable salt, derived form or composition thereof, for use as a medicament.

19. A compound of formula (1) as described in claim 1 or a pharmaceutically acceptable salt, derived form or composition thereof, for use in the treatment of diseases, disorders, and conditions in which the β2 receptor is involved.

20. A compound of formula (1) as described in claim 1 or a pharmaceutically acceptable salt, derived form or composition thereof, for use in the treatment of diseases, disorders, and conditions selected from the group consisting of :
• asthma of whatever type, etiology, or pathogenesis, in particular asthma that is a member selected from the group consisting of atopic asthma, non-atopic asthma, allergic asthma, atopic bronchial IgE-mediated asthma, bronchial asthma, essential asthma, true asthma, intrinsic asthma caused by pathophysiologic disturbances, extrinsic asthma caused by environmental factors, essential asthma of unknown or inapparent cause, non-atopic asthma, bronchitic asthma, emphysematous asthma, exercise-induced asthma, allergen induced asthma, cold air induced asthma, occupational asthma, infective asthma caused by bacterial, fungal, protozoal, or viral infection, non-allergic asthma, incipient asthma, wheezy infant syndrome and bronchiolytis,
• chronic or acute bronchoconstriction, chronic bronchitis, small airways obstruction, and emphysema,
• obstructive or inflammatory airways diseases of whatever type, etiology, or pathogenesis, in particular an obstructive or inflammatory airways disease that is a member selected from the group consisting of chronic eosinophilic pneumonia, chronic obstructive pulmonary disease (COPD), COPD that includes chronic bronchitis, pulmonary emphysema or dyspnea associated or not associated with COPD, COPD that is **characterized by** irreversible, progressive airways obstruction, adult respiratory distress syndrome (ARDS), exacerbation of airways hyper-reactivity consequent to other drug therapy and airways disease that is associated with pulmonary hypertension,
• bronchitis of whatever type, etiology, or pathogenesis, in particular bronchitis that is a member selected from the group consisting of acute bronchitis, acute laryngotracheal bronchitis, arachidic bronchitis, catarrhal bronchitis, croupus bronchitis, dry bronchitis, infectious asthmatic bronchitis, productive bronchitis, staphylococcus or streptococcal bronchitis and vesicular bronchitis,
• bronchiectasis of whatever type, etiology, or pathogenesis, in particular bronchiectasis that is a member selected from the group consisting of cylindric bronchiectasis, sacculated bronchiectasis, fusiform bronchiectasis, capillary bronchiectasis, cystic bronchiectasis, dry bronchiectasis and follicular bronchiectasis.

21. The use of a compound of formula (1) as described in claim 1 or of a pharmaceutically acceptable salt, derived form or composition thereof, for the manufacture of a drug having a β2 agonist activity.

22. The use of a compound of formula (1) as described in claim 1 or of a pharmaceutically acceptable salt, solvate or composition thereof, for the manufacture of a drug for the treatment of diseases, disorders, and conditions selected from the group as described in claim 20.

23. A method of treatment of a mammal, including a human being, with a β2 agonist including treating said mammal with an effective amount of a compound of formula (1) as described in claim 1 or with a pharmaceutically acceptable salt, derived form or composition thereof.

24. A method according to claim 23 where the disease, disorder or condition is selected from the group as described in claim 20.
